# EUROPEAN PATENT APPLICATION

(11) **EP 2 617 726 A1**
(43) Date of publication of application: **24.07.2013**
(21) Application number: 11825291.5
(22) Date of filing: 16.09.2011
(51) Int. Cl.: C07D 495/04, A61K 31/4439, A61P 3/04, A61P 3/06, A61P 3/10

(54) **DIABETES THERAPEUTIC AGENT**

(30) Priority: 17.09.2010 JP 2010208852
(71) Applicant: Takeda Pharmaceutical Company Limited, Osaka-shi, Osaka 541-0045 (JP)
(72) Inventor: OKA, Masahiro, Fujisawa, Kanagawa 251-0012 (JP); YAMADA, Yukio, Fujisawa, Kanagawa 251-0012 (JP)
(74) Representative: König, Gregor Sebastian
(86) International application number: PCT/JP2011/071285
(87) International publication number: WO 2012/036293

(57) **Abstract**

The problem of the present invention is to provide a compound useful for the prophylaxis or treatment of diseases related to the control of the differentiation of muscle cell or adipocyte differentiation, such as diabetes, obesity, dyslipidemia, and having a superior efficacy.
The present invention provides an agent for the prophylaxis or treatment of diabetes, obesity or dyslipidemia, containing a compound represented by the formula (I) 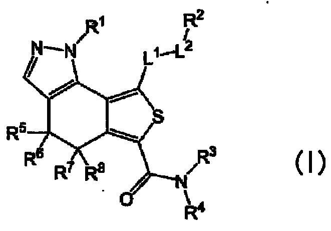 wherein each symbol is as defined in the DESCRIPTION, or a salt thereof or a prodrug thereof.

## Description

### Technical Field

The present invention relates to an agent for the prophylaxis or treatment of diseases related to the control of the differentiation of muscle cell or adipocyte, such as diabetes, obesity, dyslipidemia, which has a muscle cell and/or adipocyte differentiation regulating action, and various pharmacological actions based on promotion of differentiation of myoblast, suppression of differentiation of adipocyte, and/or inhibition of activation of NF-kB.

### Background of the Invention

Differentiation of myoblast, differentiation of adipocyte and activation of NF-kB are considered to play an important role in various diseases. For example, in diseases such as diabetes, obesity, dyslipidemia, it is considered that suppression of myoblast differentiation, promotion of adipocyte differentiation, or promotion of NF-kB activity is involved in the onset and aggravation of the diseases. In fact, obesity wherein adipose tissue amount has increased is a risk factor of diabetes, hypertension, dyslipidemia and the like, and it has been reported muscle mass decreases in diabetes and obesity. Diabetes (2006)55, 1813-1818 (non-patent document 1) describes that type 2 diabetes patients show decreased muscle mass, and Mechanisms of Ageing and Development (2003)124, 495-502 (non-patent document 2) indicates that obesity of body trunk and decrease of muscle mass are related. It has also been reported that the muscle mass increases when NF-kB pathway is suppressed by a proteasome inhibitor.
Skeletal muscles, which are present in human in not less than 600, are tissues occupying the largest proportion of the body weight, and consume sugar by insulin stimulation. On the other hand, obesity is a condition showing an increased amount of adipose tissue, and it is considered that abnormal metabolism and organ disorder occur from obese adipocyte due to insulin resistance caused by decreased adiponectin secretion and promoted secretion of inflammatory cytokines such as TNF-α and the like, and the like. Therefrom increase of muscle mass and suppression of adipocyte differentiation are considered to be useful for the improvement of glucose and lipid metabolism.
Steroid hormone is known to increase the muscle mass. However, steroid hormone is known to cause various side effects. While use of a myostatin antibody and a soluble activin receptor, which is a myostatin receptor, to increase the muscle mass is being studied, it has not been put to practical use. In addition, the suppressive action of a steroid hormone, a myostatin antibody or a soluble activin receptor on adipocyte differentiation has not been reported.
There is still a high need for a prophylactic or therapeutic drug for diabetes, obesity, dyslipidemia and the like, and the development of a strong therapeutic drug with less side effects is desired.
On the other hand, the compounds of from Reference Example 7 to Reference Example 42 of the present specification have an enhancing action for cell differentiation inducer, and are described in WO01/74823 (patent document 1) as compounds usable for the prophylaxis or treatment of bone diseases or neurological diseases.
In addition, the following compounds have been reported.

### (1) A compound represented by the formula:

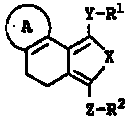

wherein X is a sulfur atom or an oxygen atom; Y is an optionally oxidized sulfur atom or oxygen atom; Z is a bond or a divalent hydrocarbon group; R¹ is an optionally substituted hydrocarbon group; R² is an optionally amidated or esterified carboxyl group; and ring A is an optionally substituted aromatic 5-membered heterocyclic ring; provided that when both X and Y are S,

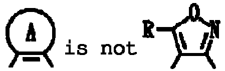

wherein R is a hydrogen atom or a C₁₋₆ alkyl group, or a salt thereof (WO98/09958: patent document 2).

### (2) A dihydrobenz[c]thiophene compound represented by the formula:

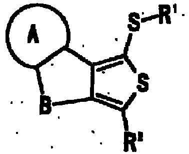

wherein R¹ is a lower alkyl group, R² is a carbamoyl group, A is an aromatic 5-membered ring containing 1 - 3, the same or different nitrogen atom, oxygen atom or sulfur atom, which is substituted by a lower alkyl group or a phenyl group, and B is a group represented by the formula -X-CH₂- (wherein X is -CR₃R₄-wherein R₃ and R₄ are each a lower alkyl group or a phenyl group), an oxygen atom or a sulfur atom, or a pharmaceutically acceptable salt thereof (JP-A-2000-309591: patent document 3).
However, it is not known that these compounds have a cell differentiation regulating action of muscle cell and/or adipocyte.

### [Document List]

### [patent documents]

patent document 1: WO01/74823
patent document 2: WO98/09958
patent document 3: JP-A-2000-309591

### [non-patent documents]

non-patent document 1: Diabetes (2006)55, 1813-1818
non-patent document 2: Mechanisms of Ageing and Development (2003)124, 495-502

### SUMMARY OF THE INVENTION

### Problems to be Solved by the Invention

An object of the present invention is to provide a compound useful for the prophylaxis or treatment of diseases related to the control of the differentiation of muscle cell or adipocyte, such as diabetes, obesity, dyslipidemia, and superior in pharmacological action, physicochemical properties and the like.

### Means of Solving the Problems

The present inventors have found for the first time that a heterocyclic compound represented by the following formula (I) or a salt thereof (hereinafter sometimes to be referred to as compound (I)) has a muscle cell and/or adipocyte differentiation regulating action, shows various pharmacological actions based on promotion of differentiation of myoblast, suppression of differentiation of adipocyte, and/or inhibition of activation of NF-kB, and is useful for the prophylaxis or treatment of diseases related to the control of the differentiation of muscle cell or adipocyte, such as diabetes, obesity, dyslipidemia. The present inventors have conducted intensive studies based on these findings and completed the present invention.

Accordingly, the present invention relates to
[1] an agent for the prophylaxis or treatment of diabetes, obesity or dyslipidemia, comprising a compound represented by the formula (I)

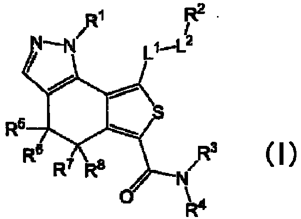

wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group,
R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof or a prodrug thereof;

[2] an agent for regulating muscle cell and/or adipocyte differentiation, comprising a compound represented by the formula (I)

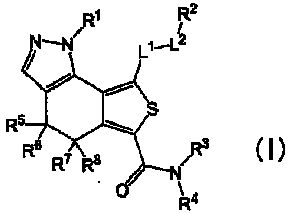

wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof or a prodrug thereof;

[3] the agent of [1] or [2], wherein R² is
   (1) a C₁₋₆ alkyl group optionally substituted by 1 to 3 substituents selected from
      (a) a C₁₋₆ alkoxy group, and
      (b) a 5- or 6-membered aromatic heterocyclic group,
   (2) a C₃₋₈ cycloalkyl group,
   (3) a 5- to 10-membered aromatic heterocyclic group optionally substituted by 1 to 3 substituents selected from
      (a) a C₁₋₆ alkyl group,
      (b) a C₁₋₆ alkoxy group optionally substituted by 1 to 3 substituents selected from
         (i) a C₁₋₆ alkoxy group,
         (ii) an amino group optionally mono- or di-substituted by substituent(s) selected from a C₁₋₆ alkyl group and a phenyl group, and
         (iii) a 5- or 6-membered nonaromatic heterocyclic group optionally substituted by an oxo group,
      (c) an amino group, and
      (d) an oxide group (-O⁻), or
   (4) a phenyl group optionally substituted by 1 to 3 substituents selected from
      (a) an amino group optionally mono- or di-substituted by substituent(s) selected from
         (i) a C₁₋₆ alkyl group, and
         (ii) a C₁₋₆ alkyl-carbonyl group optionally substituted by 1 to 3 halogen atoms,
      (b) a C₁₋₆ alkylsulfonyl group,
      (c) a C₁₋₆ alkoxy group optionally substituted by 1 to 3 substituents selected from
         (i) a halogen atom,
         (ii) a 5- to 10-membered aromatic heterocyclic group, and
         (iii) a carboxy group (-COOH),
      (d) a halogen atom,
      (e) a C₁₋₆ alkyl group optionally substituted by 1 to 3 substituents selected from
         (i) a carboxy group,
         (ii) an oxo group,
         (iii) a 5- or 6-membered nonaromatic heterocyclic group, and
         (iv) an amino group optionally mono- or di-substituted by C₁₋₆ alkyl group(s), and
      (f) a C₁₋₃ alkylenedioxy group;
[4] the agent of any of [1] to [3], wherein R³ is
   (1) a hydrogen atom,
   (2) a C₁₋₆ alkyl group optionally substituted by 1 to 3 5- or 6-membered aromatic heterocyclic groups, or
   (3) a 5- or 6-membered aromatic heterocyclic group;
[5] the agent of any of [1] to [4], wherein R¹ is a hydrogen atom or methyl;
   R² is
   (1) a C₁₋₆ alkyl group optionally substituted by 1 to 3 substituents selected from
      (a) a C₁₋₆ alkoxy group, and
      (b) a 5- or 6-membered aromatic heterocyclic group,
   (2) a C₃₋₈ cycloalkyl group,
   (3) a 5- to 10-membered aromatic heterocyclic group optionally substituted by 1 to 3 substituents selected from
      (a) a C₁₋₆ alkyl group,
      (b) a C₁₋₆ alkoxy group optionally substituted by 1 to 3 substituents selected from
         (i) a C₁₋₆ alkoxy group,
         (ii) an amino group optionally mono- or di-substituted by substituent(s) selected from a C₁₋₆ alkyl group and a phenyl group, and
         (iii) a 5- or 6-membered nonaromatic heterocyclic group optionally substituted by an oxo group,
      (c) an amino group, and
      (d) an oxide group (-O⁻), or
   (4) a phenyl group optionally substituted by 1 to 3 substituents selected from
      (a) an amino group optionally mono- or di-substituted by substituent(s) selected from
         (i) a C₁₋₆ alkyl group, and
         (ii) a C₁₋₆ alkyl-carbonyl group optionally substituted by 1 to 3 halogen atoms,
      (b) a C₁₋₆ alkylsulfonyl group,
      (c) a C₁₋₆ alkoxy group optionally substituted by 1 to 3 substituents selected from
         (i) a halogen atom,
         (ii) a 5- to 10-membered aromatic heterocyclic group, and
         (iii) a carboxy group,
      (d) a halogen atom,
      (e) a C₁₋₆ alkyl group optionally substituted by 1 to 3 substituents selected from
         (i) a carboxy group,
         (ii) an oxo group,
         (iii) a 5- or 6-membered nonaromatic heterocyclic group, and
         (iv) an amino group optionally mono- or di-substituted by C₁₋₆ alkyl group(s), and
      (f) a C₁₋₃ alkylenedioxy group;
   R³ is
   (1) a hydrogen atom,
   (2) a C₁₋₆ alkyl group optionally substituted by 1 to 3 5- or 6-membered aromatic heterocyclic groups, or
   (3) a 5- or 6-membered aromatic heterocyclic group;
   R⁴ is a hydrogen atom;
   R⁵ is a hydrogen atom or methyl;
   R⁶ is a hydrogen atom or methyl;
   R⁷ is a hydrogen atom;
   R⁸ is a hydrogen atom;
   L¹ is a bond, O, S, SO₂ or NR⁹;
   R⁹ is a hydrogen atom or a C₁₋₆ alkyl group; and
   L² is a bond or methylene;
[6] a method for the prophylaxis or treatment of diabetes, obesity or dyslipidemia in a mammal, comprising administering an effective amount of a compound represented by the formula (I)

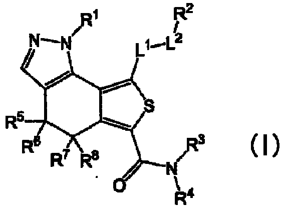

wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group,
R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof or a prodrug thereof to said mammal;
[7] a method of regulating muscle cell and/or adipocyte differentiation in a mammal, comprising administering an effective amount of a compound represented by the formula (I)

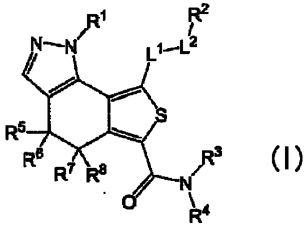

wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof or a prodrug thereof to said mammal;
[8] use of a compound represented by the formula (I)

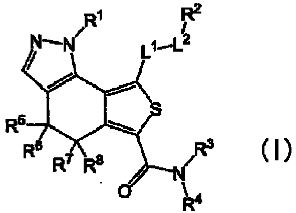

wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof or a prodrug thereof for producing an agent for the prophylaxis or treatment of diabetes, obesity or dyslipidemia;
[9] use of a compound represented by the formula (I)

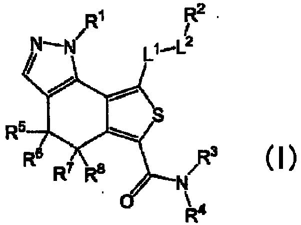

wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof or a prodrug thereof for producing an agent for regulating muscle cell and/or adipocyte differentiation;
[10] a compound represented by the formula (I):

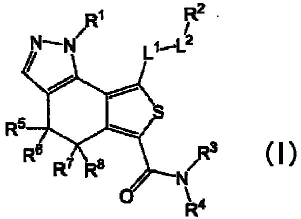

wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof or a prodrug thereof for the prophylaxis or treatment of diabetes, obesity or dyslipidemia;
[11] a compound represented by the formula (I):

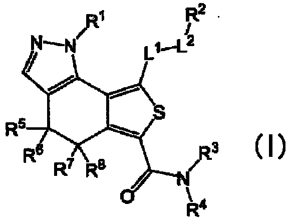

wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof or a prodrug thereof for regulating muscle cell and/or adipocyte differentiation; and the like.

### Effect of the Invention

The below-mentioned compound (II) has an action to promote differentiation of muscle cell and/or suppress differentiation of adipocyte, and improve glucose and lipid metabolism, and is useful for the prophylaxis or treatment of diseases showing abnormal glucose and lipid metabolism function of tissues, and diseases associated with decreased muscle mass and/or increased adipose tissue.

### Description of Embodiments

### (Detailed Description of the Invention)

The definition of each symbol in the formula (I) is described in detail in the following.

Unless otherwise specified, the "halogen atom" in the present specification means a fluorine atom, a chlorine atom, a bromine atom and an iodine atom.

Unless otherwise specified, the "C₁₋₆ alkyl group" in the present specification means methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like.

Unless otherwise specified, the "C₁₋₆ alkoxy group" in the present specification means methoxy, ethoxy, propoxy, isopropoxy, butoxy, isobutoxy, sec-butoxy, tert-butoxy and the like.

Unless otherwise specified, the "C₁₋₆ alkyl-carbonyl group" in the present specification means acetyl, propanoyl, butanoyl, isobutanoyl, pentanoyl, isopentanoyl, hexanoyl and the like.

Unless otherwise specified, the "C₁₋₆ alkoxy-carbonyl group" in the present specification means methoxycarbonyl, ethoxycarbonyl, propoxycarbonyl, isopropoxycarbonyl, tert-butoxycarbonyl and the like.

Unless otherwise specified, the "C₃₋₈ cycloalkyl group" in the present specification means cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl and the like.

R¹ is a hydrogen atom or a C₁₋₆ alkyl group.

R¹ is preferably a hydrogen atom or methyl.

R² is a hydrogen atom, an optionally substituted C₁₋₆ alkyl group, an optionally substituted C₃₋₈ cycloalkyl group, an optionally substituted aromatic heterocyclic group or an optionally substituted phenyl group.

The C₁₋₆ alkyl group of the "optionally substituted C₁₋₆ alkyl group" for R² optionally has 1 to 3 substituents at substitutable positions.

Examples of said substituent include
(1) a C₃₋₁₀ cycloalkyl group (e.g., cyclopropyl, cyclohexyl);
(2) a C₆₋₁₄ aryl group (e.g., phenyl, naphthyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(3) a 4- to 7-membered aromatic heterocyclic group (e.g., thienyl, furyl, pyridyl, pyrazolyl, imidazolyl, tetrazolyl, oxazolyl, thiazolyl, oxadiazolyl, thiadiazolyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(4) a 4- to 7-membered nonaromatic heterocyclic group (e.g., tetrahydrofuryl, morpholinyl, thiomorpholinyl, piperidinyl, pyrrolidinyl, piperazinyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms,
   (d) a halogen atom, and
   (e) an oxo group;
(5) an amino group optionally mono- or di-substituted by substituent(s) selected from
   (a) a C₁₋₆ alkyl group optionally substituted by 1 to 3 halogen atoms,
   (b) a C₁₋₆ alkyl-carbonyl group optionally substituted by 1 to 3 halogen atoms,
   (c) a C₁₋₆ alkoxy-carbonyl group optionally substituted by 1 to 3 halogen atoms,
   (d) a C₁₋₆ alkylsulfonyl group (e.g., methylsulfonyl) optionally substituted by 1 to 3 halogen atoms,
   (e) a carbamoyl group optionally mono- or di-substituted by C₁₋₆ alkyl group(s) optionally substituted by 1 to 3 halogen atoms, and
   (f) a 4- to 7-membered aromatic heterocyclic group (e.g., thienyl, furyl, pyridyl, pyrazolyl, imidazolyl, tetrazolyl, oxazolyl, thiazolyl, oxadiazolyl, thiadiazolyl);
(6) a C₁₋₆ alkyl-carbonyl group optionally substituted by 1 to 3 halogen atoms;
(7) a C₁₋₆ alkoxy-carbonyl group (e.g., methoxycarbonyl) optionally substituted by 1 to 3 substituents selected from
   (a) a halogen atom,
   (b) a C₁₋₆ alkoxy group,
   (c) a C₆₋₁₄ aryl group (e.g., phenyl), and
   (d) a 4- to 7-membered heterocyclic group (preferably a 4- to 7-membered nonaromatic heterocyclic group (e.g., tetrahydrofuryl));
(8) a C₁₋₆ alkylsulfonyl group (e.g., methylsulfonyl, ethylsulfonyl, isopropylsulfonyl) optionally substituted by 1 to 3 halogen atoms;
(9) a carbamoyl group optionally mono- or di-substituted by C₁₋₆ alkyl group(s) optionally substituted by 1 to 3 halogen atoms;
(10) a thiocarbamoyl group optionally mono- or di-substituted by C₁₋₆ alkyl group(s) optionally substituted by 1 to 3 halogen atoms;
(11) a sulfamoyl group optionally mono- or di-substituted by C₁₋₆ alkyl group(s) optionally substituted by 1 to 3 halogen atoms;
(12) a carboxy group;
(13) a hydroxy group;
(14) a C₁₋₆ alkoxy group optionally substituted by 1 to 3 substituents selected from
   (a) a halogen atom,
   (b) a carboxy group,
   (c) a C₁₋₆ alkoxy group,
   (d) a C₁₋₆ alkoxy-carbonyl group optionally substituted by 1 to 3 C₆₋₁₄ aryl groups (e.g., phenyl),
   (e) an amino group optionally mono- or di-substituted by substituent(s) selected from a C₁₋₆ alkyl group and a C₁₋₆ alkoxy-carbonyl group,
   (f) a 4- to 7-membered heterocyclic group (preferably a 4- to 7-membered nonaromatic heterocyclic group (e.g., tetrahydrofuryl)), and
   (g) a C₃₋₁₀ cycloalkyl group;
(15) a C₂₋₆ alkenyloxy group (e.g., ethenyloxy) optionally substituted by 1 to 3 halogen atoms;
(16) a C₇₋₁₃ aralkyloxy group (e.g., benzyloxy);
(17) a C₆₋₁₄ aryloxy group (e.g., phenyloxy, naphthyloxy);
(18) a C₁₋₆ alkyl-carbonyloxy group (e.g., acetyloxy, tert-butylcarbonyloxy);
(19) a C₆₋₁₄ aryl-carbonyl group (e.g., benzoyl) optionally substituted by 1 to 3 substituents selected from
   (a) a halogen atom, and
   (b) a C₁₋₆ alkyl group optionally substituted by 1 to 3 halogen atoms;
(20) a 4- to 7-membered nonaromatic heterocyclylcarbonyl group (e.g., pyrrolidinylcarbonyl, morpholinylcarbonyl) optionally substituted by 1 to 3 substituents selected from a C₁₋₆ alkyl group optionally substituted by 1 to 3 halogen atoms;
(21) a mercapto group;
(22) a C₁₋₆ alkylthio group (e.g., methylthio, ethylthio) optionally substituted by 1 to 3 substituents selected from
   (a) a halogen atom, and
   (b) a C₁₋₆ alkoxy-carbonyl group;
(23) a C₇₋₁₃ aralkylthio group (e.g., benzylthio);
(24) a C₆₋₁₄ arylthio group (e.g., phenylthio, naphthylthio);
(25) a cyano group;
(26) a nitro group;
(27) a halogen atom (e.g., a fluorine atom, a chlorine atom, a bromine atom);
(28) a C₁₋₃ alkylenedioxy group;
(29) a C₁₋₃ alkyleneoxy group (e.g., methyleneoxy, ethyleneoxy);
(30) a 4- to 7-membered aromatic heterocyclylcarbonyl group (e.g., pyrazolylcarbonyl, pyrazinylcarbonyl, isoxazolylcarbonyl, pyridylcarbonyl, thiazolylcarbonyl) optionally substituted by 1 to 3 substituents selected from a C₁₋₆ alkyl group optionally substituted by 1 to 3 halogen atoms;
(31) a C₃₋₁₀ cycloalkoxy group (e.g., cyclopropoxy, cyclopentyloxy) optionally substituted by 1 to 3 substituents selected from
   (a) a halogen atom (e.g., a fluorine atom), and
   (b) a C₁₋₆ alkoxy group (e.g., methoxy);
and the like. When the number of the substituents is two or more, the respective substituents may be the same or different.

The "optionally substituted C₁₋₆ alkyl group" for R² is preferably a C₁₋₆ alkyl group optionally substituted by 1 to 3 substituents selected from
(1) a C₁₋₆ alkoxy group; and
(2) a 5- or 6-membered aromatic heterocyclic group (e.g., pyridyl);
   and the like. When the number of the substituents is two or more, the respective substituents may be the same or different.

Examples of the "aromatic heterocyclic group" of the "optionally substituted aromatic heterocyclic group" for R² include a 4- to 7-membered (preferably 5- or 6-membered) monocyclic aromatic heterocyclic group containing, as a ring-constituting atom besides carbon atom, 1 to 4 hetero atoms selected from an oxygen atom, a sulfur atom and a nitrogen atom, and a fused aromatic heterocyclic group. Examples of said fused aromatic heterocyclic group include a group derived from a fused ring of a ring corresponding to such 4- to 7-membered monocyclic aromatic heterocyclic group and 1 or 2 selected from a 5- or 6-membered aromatic heterocycle (e.g., pyrrole, imidazole, pyrazole, pyrazine, pyridine, pyrimidine) containing 1 or 2 nitrogen atoms, a 5-membered aromatic heterocycle (e.g., thiophene) containing one sulfur atom, and a benzene ring, and the like.

Preferable examples of the aromatic heterocyclic group include 5- to 10-membered aromatic heterocyclic groups such as monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 3-isothiazolyl, 4-isothiazolyl, 5-isothiazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazin-1-yl, 1,2,4-triazin-3-yl); fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuranyl (e.g., 2-benzofuranyl, 3-benzofuranyl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridyl (e.g., 1H-imidazo[4,5-b]pyridin-2-yl, 1H-imidazo[4,5-c]pyridin-2-yl, 2H-imidazo[1,2-a]pyridin-3-yl), thienopyridyl (e.g., thieno[2,3-b]pyridin-3-yl), imidazopyrazinyl (e.g., 1H-imidazo[4,5-b]pyrazin-2-yl), pyrazolopyridyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl), pyridopyridyl (e.g., pyrido[2,3-b]pyridin-3-yl), thienopyridyl (e.g., thieno[2,3-b]pyridin-3-yl).

The C₃₋₈ cycloalkyl group, aromatic heterocyclic group and phenyl group of the "optionally substituted C₃₋₈ cycloalkyl group", "optionally substituted aromatic heterocyclic group" and "optionally substituted phenyl group" for R² optionally have 1 to 3 substituents at substitutable positions.

Examples of said substituent include
(1) a C₃₋₁₀ cycloalkyl group (e.g., cyclopropyl, cyclopentyl, cyclohexyl);
(2) a C₆₋₁₄ aryl group (e.g., phenyl, naphthyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(3) a 4- to 7-membered aromatic heterocyclic group (e.g., thienyl, furyl, pyridyl, pyrazolyl, imidazolyl, tetrazolyl, oxazolyl, thiazolyl, oxadiazolyl, thiadiazolyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms, and
   (d) a halogen atom;
(4) a 4- to 7-membered nonaromatic heterocyclic group (e.g., tetrahydrofuryl, morpholinyl, thiomorpholinyl, piperidinyl, pyrrolidinyl, piperazinyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group optionally substituted by 1 to 3 halogen atoms,
   (d) a halogen atom, and
   (e) an oxo group;
(5) an amino group optionally mono- or di-substituted by substituent(s) selected from
   (a) a C₁₋₆ alkyl group optionally substituted by 1 to 3 halogen atoms,
   (b) a C₁₋₆ alkyl-carbonyl group optionally substituted by 1 to 3 halogen atoms,
   (c) a C₁₋₆ alkoxy-carbonyl group optionally substituted by 1 to 3 halogen atoms,
   (d) a C₁₋₆ alkylsulfonyl group (e.g., methylsulfonyl) optionally substituted by 1 to 3 halogen atoms,
   (e) a carbamoyl group optionally mono- or di-substituted by C₁₋₆ alkyl group(s) optionally substituted by 1 to 3 halogen atoms, and
   (f) a 4- to 7-membered aromatic heterocyclic group (e.g., thienyl, furyl, pyridyl, pyrazolyl, imidazolyl, tetrazolyl, oxazolyl, thiazolyl, oxadiazolyl, thiadiazolyl);
(6) a C₁₋₆ alkyl-carbonyl group optionally substituted by 1 to 3 halogen atoms;
(7) a C₁₋₆ alkoxy-carbonyl group (e.g., methoxycarbonyl) optionally substituted by 1 to 3 substituents selected from
   (a) a halogen atom,
   (b) a C₁₋₆ alkoxy group,
   (c) a C₆₋₁₄ aryl group (e.g., phenyl), and
   (d) a 4- to 7-membered heterocyclic group (preferably a 4- to 7-membered nonaromatic heterocyclic group (e.g., tetrahydrofuryl));
(8) a C₁₋₆ alkylsulfonyl group (e.g., methylsulfonyl, ethylsulfonyl, isopropylsulfonyl) optionally substituted by 1 to 3 halogen atoms;
(9) a carbamoyl group optionally mono- or di-substituted by C₁₋₆ alkyl group(s) optionally substituted by 1 to 3 halogen atoms;
(10) a thiocarbamoyl group optionally mono- or di-substituted by C₁₋₆ alkyl group(s) optionally substituted by 1 to 3 halogen atoms;
(11) a sulfamoyl group optionally mono- or di-substituted by C₁₋₆ alkyl group(s) optionally substituted by 1 to 3 halogen atoms;
(12) a carboxy group;
(13) a hydroxy group;
(14) a C₁₋₆ alkoxy group optionally substituted by 1 to 3 substituents selected from
   (a) a halogen atom,
   (b) a carboxy group,
   (c) a C₁₋₆ alkoxy group,
   (d) a C₁₋₆ alkoxy-carbonyl group optionally substituted by 1 to 3 C₆₋₁₄ aryl groups (e.g., phenyl),
   (e) an amino group optionally mono- or di-substituted by substituent(s) selected from a C₁₋₆ alkyl group, a phenyl group and a C₁₋₆ alkoxy-carbonyl group,
   (f) a 5- to 10-membered heterocyclic group (e.g., quinolyl, pyridyl, dihydroquinolyl, dihydropyridyl) optionally substituted by 1 to 3 oxo groups, and
   (g) a C₃₋₁₀ cycloalkyl group;
(15) a C₂₋₆ alkenyloxy group (e.g., ethenyloxy) optionally substituted by 1 to 3 halogen atoms;
(16) a C₇₋₁₃ aralkyloxy group (e.g., benzyloxy);
(17) a C₆₋₁₄ aryloxy group (e.g., phenyloxy, naphthyloxy);
(18) a C₁₋₆ alkyl-carbonyloxy group (e.g., acetyloxy, tert-butylcarbonyloxy);
(19) a C₆₋₁₄ aryl-carbonyl group (e.g., benzoyl) optionally substituted by 1 to 3 substituents selected from
   (a) a halogen atom, and
   (b) a C₁₋₆ alkyl group optionally substituted by 1 to 3 halogen atoms;
(20) a 4- to 7-membered nonaromatic heterocyclylcarbonyl group (e.g., pyrrolidinylcarbonyl, morpholinylcarbonyl) optionally substituted by 1 to 3 substituents selected from a C₁₋₆ alkyl group optionally substituted by 1 to 3 halogen atoms;
(21) a mercapto group;
(22) a C₁₋₆ alkylthio group (e.g., methylthio, ethylthio) optionally substituted by 1 to 3 substituents selected from
   (a) a halogen atom, and
   (b) a C₁₋₆ alkoxy-carbonyl group;
(23) a C₇₋₁₃ aralkylthio group (e.g., benzylthio);
(24) a C₆₋₁₄ arylthio group (e.g., phenylthio, naphthylthio);
(25) a cyano group;
(26) a nitro group;
(27) a halogen atom (e.g., a fluorine atom, a chlorine atom, a bromine atom);
(28) a C₁₋₃ alkylenedioxy group (e.g., methylenedioxy);
(29) a C₁₋₃ alkyleneoxy group (e.g., methyleneoxy, ethyleneoxy);
(30) a 4- to 7-membered aromatic heterocyclylcarbonyl group (e.g., pyrazolylcarbonyl, pyrazinylcarbonyl, isoxazolylcarbonyl, pyridylcarbonyl, thiazolylcarbonyl) optionally substituted by 1 to 3 substituents selected from a C₁₋₆ alkyl group optionally substituted by 1 to 3 halogen atoms;
(31) a C₃₋₁₀ cycloalkoxy group (e.g., cyclopropoxy, cyclopentyloxy) optionally substituted by 1 to 3 substituents selected from
   (a) a halogen atom (e.g., fluorine atom), and
   (b) a C₁₋₆ alkoxy group (e.g., methoxy);
(32) a C₁₋₆ alkyl group optionally substituted by 1 to 3 substituents selected from
   (a) a halogen atom,
   (b) a carboxy group,
   (c) a hydroxy group,
   (d) a C₁₋₆ alkoxy-carbonyl group,
   (e) a C₁₋₆ alkoxy group,
   (f) an amino group optionally mono- or di-substituted by C₁₋₆ alkyl group(s),
   (g) a 4- to 7-membered heterocyclic group (preferably a 5- or 6-membered nonaromatic heterocyclic group (e.g., morpholinyl)), and
   (h) an oxo group;
(33) a C₂₋₆ alkenyl group (e.g., ethenyl, 1-propenyl) optionally substituted by 1 to 3 substituents selected from
   (a) a halogen atom,
   (b) a carboxy group,
   (c) a hydroxy group,
   (d) a C₁₋₆ alkoxy-carbonyl group,
   (e) a C₁₋₆ alkoxy group, and
   (f) an amino group optionally mono- or di-substituted by C₁₋₆ alkyl group(s);
(34) a C₇₋₁₃ aralkyl group (e.g., benzyl) optionally substituted by 1 to 3 substituents selected from
   (a) a C₁₋₆ alkyl group optionally substituted by 1 to 3 halogen atoms,
   (b) a hydroxy group,
   (c) a C₁₋₆ alkoxy group, and
   (d) a halogen atom;
(35) an oxo group;
(36) an oxide group (-O⁻);
and the like. When the number of the substituents is two or more, the respective substituents may be the same or different.

The "optionally substituted C₃₋₈ cycloalkyl group" for R² is preferably a C₃₋₈ cycloalkyl group and the like.

The "optionally substituted aromatic heterocyclic group" for R² is preferably a 5- to 10-membered aromatic heterocyclic group optionally substituted by 1 to 3 substituents selected from
(1) a C₁₋₆ alkyl group;
(2) a C₁₋₆ alkoxy group optionally substituted by 1 to 3 substituents selected from
   (a) a C₁₋₆ alkoxy group,
   (b) an amino group optionally mono- or di-substituted by substituent(s) selected from a C₁₋₆ alkyl group and a phenyl group, and
   (c) a 5- or 6-membered nonaromatic heterocyclic group (e.g., pyrrolidinyl) optionally substituted by an oxo group;
(3) an amino group;
(4) an oxide group (-O⁻);
   and the like. When the number of the substituents is two or more, the respective substituents may be the same or different.

The "optionally substituted phenyl group" for R² is preferably a phenyl group optionally substituted by 1 to 3 substituents selected from
(1) an amino group optionally mono- or di-substituted by substituent(s) selected from
   (a) a C₁₋₆ alkyl group, and
   (b) a C₁₋₆ alkyl-carbonyl group optionally substituted by 1 to 3 halogen atoms;
(2) a C₁₋₆ alkylsulfonyl group (e.g., methylsulfonyl, ethylsulfonyl, isopropylsulfonyl);
(3) a C₁₋₆ alkoxy group optionally substituted by 1 to 3 substituents selected from
   (a) a halogen atom, and
   (b) a 5- to 10-membered aromatic heterocyclic group (e.g., quinolyl, pyridyl),
   (c) a carboxy group;
(4) a halogen atom (e.g., a fluorine atom, a chlorine atom, a bromine atom);
(5) a C₁₋₆ alkyl group optionally substituted by 1 to 3 substituents selected from
   (a) a carboxy group,
   (b) an oxo group,
   (c) a 5- or 6-membered nonaromatic heterocyclic group (e.g., morpholinyl), and
   (d) an amino group optionally mono- or di-substituted by C₁₋₆ alkyl group(s);
(6) a C₁₋₃ alkylenedioxy group (e.g., methylenedioxy);
and the like.

R² is preferably
(1) a C₁₋₆ alkyl group optionally substituted by 1 to 3 substituents selected from
   (a) a C₁₋₆ alkoxy group, and
   (b) a 5- or 6-membered aromatic heterocyclic group (e.g., pyridyl);
(2) a C₃₋₈ cycloalkyl group (e.g., cyclopentyl);
(3) a 5- to 10-membered aromatic heterocyclic group (e.g., quinolyl) optionally substituted by 1 to 3 substituents selected from
   (a) a C₁₋₆ alkyl group,
   (b) a C₁₋₆ alkoxy group optionally substituted by 1 to 3 substituents selected from
      (i) a C₁₋₆ alkoxy group,
      (ii) an amino group optionally mono- or di-substituted by substituent(s) selected from a C₁₋₆ alkyl group and a phenyl group, and
      (iii) a 5- or 6-membered nonaromatic heterocyclic group (e.g., pyrrolidinyl) optionally substituted by an oxo group,
   (c) an amino group, and
   (d) an oxide group (-O⁻);
(4) a phenyl group optionally substituted by 1 to 3 substituents selected from
   (a) an amino group optionally mono- or di-substituted by substituent(s) selected from
      (i) a C₁₋₆ alkyl group, and
      (ii) a C₁₋₆ alkyl-carbonyl group optionally substituted by 1 to 3 halogen atoms,
   (b) a C₁₋₆ alkylsulfonyl group (e.g., methylsulfonyl, ethylsulfonyl, isopropylsulfonyl),
   (c) a C₁₋₆ alkoxy group optionally substituted by 1 to 3 substituents selected from
      (i) a halogen atom, and
      (ii) a 5- to 10-membered aromatic heterocyclic group (e.g., quinolyl, pyridyl)
      (iii) a carboxy group,
   (d) a halogen atom (e.g., a fluorine atom, a chlorine atom, a bromine atom),
   (e) a C₁₋₆ alkyl group optionally substituted by 1 to 3 substituents selected from
      (i) a carboxy group,
      (ii) an oxo group,
      (iii) a 5- or 6-membered nonaromatic heterocyclic group (e.g., morpholinyl), and
      (iv) an amino group optionally mono- or di-substituted by C₁₋₆ alkyl group(s), and
   (f) a C₁₋₃ alkylenedioxy group (e.g., methylenedioxy); and the like. When the number of the substituents is two or more, the respective substituents may be the same or different.

R³ is a hydrogen atom, an optionally substituted C₁₋₆ alkyl group or an optionally substituted aromatic heterocyclic group.

The "optionally substituted C₁₋₆ alkyl group" for R³ optionally has 1 to 3 substituents at substitutable positions. Examples of said substituent include those similar to the substituent that the "C₁₋₆ alkyl group" of the "optionally substituted C₁₋₆ alkyl group" for R² has. When the number of the substituents is two or more, the respective substituents may be the same or different.
The "optionally substituted C₁₋₆ alkyl group" for R³ is preferably a C₁₋₆ alkyl group optionally substituted by 1 to 3 5- or 6-membered aromatic heterocycles (e.g., pyridyl) and the like.

Examples of the "optionally substituted aromatic heterocyclic group" for R³ include those similar to the "optionally substituted aromatic heterocyclic group" for R².

R³ is preferably
(1) a hydrogen atom,
(2) a C₁₋₆ alkyl group optionally substituted by 1 to 3 5- or 6-membered aromatic heterocycles (e.g., pyridyl),
(3) a 5- or 6-membered aromatic heterocyclic group (e.g., isoxazolyl)
or the like.

R⁴, R⁵, R⁶, R⁷ and R⁸ are each a hydrogen atom or an optionally substituted C₁₋₆ alkyl group.

Examples of the "optionally substituted C₁₋₆ alkyl group" for R⁴, R⁵, R⁶, R⁷ or R⁸ include those similar to the "optionally substituted C₁₋₆ alkyl group" for R². When the number of the substituents is two or more, the respective substituents may be the same or different.

R⁴ is preferably a hydrogen atom or the like.
R⁵ is preferably a hydrogen atom, methyl or the like.
R⁶ is preferably a hydrogen atom, methyl or the like.
R⁷ is preferably a hydrogen atom or the like.
R⁸ is preferably a hydrogen atom or the like.

L¹ is a bond, O, S, SO, SO₂ or NR⁹.

R⁹ is a hydrogen atom or an optionally substituted C₁₋₆ alkyl group.

Examples of the "optionally substituted C₁₋₆ alkyl group" for R⁹ include those similar to the "optionally substituted C₁₋₆ alkyl group" for R². When the number of the substituents is two or more, the respective substituents may be the same or different.

R⁹ is preferably a hydrogen atom, a C₁₋₆ alkyl group or the like.

L² is a bond or a C₁₋₆ alkylene group.

L² is preferably a bond, methylene or the like.

Preferable examples of compound (I) include the following compounds.

### [Compound A]

Compound (I) wherein
R¹ is a hydrogen atom or methyl;
R² is
(1) a C₁₋₆ alkyl group optionally substituted by 1 to 3 substituents selected from
   (a) a C₁₋₆ alkoxy group, and
   (b) a 5- or 6-membered aromatic heterocyclic group (e.g., pyridyl),
(2) a C₃₋₈ cycloalkyl group,
(3) a 5- to 10-membered aromatic heterocyclic group (e.g., quinolyl) optionally substituted by 1 to 3 substituents selected from
   (a) a C₁₋₆ alkyl group,
   (b) a C₁₋₆ alkoxy group optionally substituted by 1 to 3 substituents selected from
      (i) a C₁₋₆ alkoxy group,
      (ii) an amino group optionally mono- or di-substituted by substituent(s) selected from a C₁₋₆ alkyl group and a phenyl group, and
      (iii) a 5- or 6-membered nonaromatic heterocyclic group (e.g., pyrrolidinyl) optionally substituted by an oxo group,
   (c) an amino group, and
   (d) an oxide group (-O⁻), or
(4) a phenyl group optionally substituted by 1 to 3 substituents selected from
   (a) an amino group optionally mono- or di-substituted by substituent(s) selected from
      (i) a C₁₋₆ alkyl group, and
      (ii) a C₁₋₆ alkyl-carbonyl group optionally substituted by 1 to 3 halogen atoms,
   (b) a C₁₋₆ alkylsulfonyl group (e.g., methylsulfonyl, ethylsulfonyl, isopropylsulfonyl),
   (c) a C₁₋₆ alkoxy group optionally substituted by 1 to 3 substituents selected from
      (i) a halogen atom, and
      (ii) a 5- to 10-membered aromatic heterocyclic group (e.g., quinolyl, pyridyl),
      (iii) a carboxy group,
   (d) a halogen atom (e.g., a fluorine atom, a chlorine atom, a bromine atom),
   (e) a C₁₋₆ alkyl group optionally substituted by 1 to 3 substituents selected from
      (i) a carboxy group,
      (ii) an oxo group,
      (iii) a 5- or 6-membered nonaromatic heterocyclic group (e.g., morpholinyl), and
      (iv) an amino group optionally mono- or di-substituted by C₁₋₆ alkyl group(s), and
   (f) a C₁₋₃ alkylenedioxy group (e.g., methylenedioxy);
R³ is
(1) a hydrogen atom,
(2) a C₁₋₆ alkyl group optionally substituted by 1 to 3 5- or 6-membered aromatic heterocyclic groups (e.g., pyridyl), or
(3) a 5- or 6-membered aromatic heterocyclic group (e.g., isoxazolyl);
   R⁴ is a hydrogen atom,
   R⁵ is a hydrogen atom or methyl,
   R⁶ is a hydrogen atom or methyl,
   R⁷ is a hydrogen atom,
   R⁸ is a hydrogen atom,
   L¹ is a bond, O, S, SO₂ or NR⁹;
   R⁹ is a hydrogen atom or a C₁₋₆ alkyl group;
   L² is a bond or methylene.

As a salt of the compound represented by the formula (I), a pharmacologically acceptable salt is preferable. Examples of such salt include salts with inorganic bases, salts with organic bases, salts with inorganic acids, salts with organic acids, salts with basic or acidic amino acids and the like.
Preferable examples of the salts with inorganic bases include alkali metal salts such as sodium salt, potassium salt; alkaline earth metal salts such as calcium salts, magnesium salts; aluminum salts; ammonium salts, and the like.
Preferable examples of the salts with organic bases include salts with trimethylamine, triethylamine, pyridine, picoline, ethanolamine, diethanolamine, triethanolamine, dicyclohexylamine, N,N-dibenzylethylenediamine and the like.
Preferable examples of the salts with inorganic acids include salts with hydrochloric acid, hydrobromic acid, nitric acid, sulfuric acid, phosphoric acid and the like.
Preferable examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of the salts with basic amino acids include salts with arginine, lysine, ornithine and the like.
Preferable examples of the salts with acidic amino acids include salts with aspartic acid, glutamic acid and the like.

Compound (I) can be produced by a method known per se. Examples of such method include the method described in WO2004/039365, a method analogous thereto and the like.

Compound (I) may also be used as a prodrug. A prodrug of compound (I) means a compound which is converted to compound (I) with a reaction due to an enzyme or an gastric acid, etc. under the physiological condition in the living body, that is, a compound which is converted to compound (I) with enzymatic oxidation, reduction, hydrolysis and the like; a compound which is converted to compound (I) by hydrolysis and the like due to gastric acid and the like. A prodrug of compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation etc.); a compound obtained by subjecting a hydroxy group in compound (I) to an acylation, alkylation, phosphorylation or boration (e.g., a compound obtained by subjecting an hydroxy group in compound (I) to an acetylation, palmitoylation, propanoylation, pivaloylation, succinylation, fumarylation, alanylation, dimethylaminomethylcarbonylation etc.); a compound obtained by subjecting a carboxy group in compound (I) to an esterification or amidation (e.g., a compound obtained by subjecting a carboxy group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification, methylamidation etc.) and the like. These compounds can be produced from compound (I) according to a method known *per se.*
A prodrug of compound (I) may also be one which is converted into compound (I) under a physiological condition, such as those described in IYAKUHIN NO KAIHATSU (Development of Pharmaceuticals), Vol. 7, Design of Molecules, p.163-198, Published by HIROKAWA SHOTEN (1990).

The compound (I) may be labeled with an isotope (e.g., ³H, ¹¹C, ¹⁴C, ¹⁸F, ³⁵S, ¹²⁵I etc.) and the like.
The compound (I) may be a non-solvate (e.g., anhydride) or a solvate (e.g., hydrate).
Furthermore, a deuterium converter wherein ¹H is converted to ²H(D) is also encompassed in compound (I).

When compound (I) contains an optical isomer, a stereoisomer, a regioisomer and a rotamer, these are also included in compound (I), as well as each can be obtained as a single product by a synthetic method or separation method known per se. For example, when compound (I) contains an optical isomer, an optical isomer resolved from the compound is also encompassed in compound (I).

The compound (I) may also be a crystal.
The crystal of compound (I) can be produced by crystallizing compound (I) by applying a crystallization method known per se.
In the present specification, the melting point means, for example, a melting point measured by a micro melting point measurement device (Yanaco, MP-500D type or Buchi, B-545 type) or DSC (Differential scanning calorimetry) apparatus (SEIKO, EXSTAR6000) and the like.
Generally, the melting point sometimes varies depending on the measurement device, measurement condition and the like. The crystal in the present specification may be a crystal showing a melting point different from the values described in the present specification as long as the difference is within a general error range.
The compound (I) may be a pharmaceutically acceptable cocrystal or cocrystal salt. Here, the cocrystal or cocrystal salt means a crystalline substance constituted by two or more kinds of distinct solids each having different physical properties (e.g., structure, melting point, melting heat and the like) at room temperature. The cocrystal or cocrystal salt can be produced by a cocrystallization method known per se.
The crystal of compound (I) is superior in the physicochemical properties (e.g., melting point, solubility, stability) and biological properties (e.g., in vivo kinetics (absorbability, distribution, metabolism, excretion), efficacy expression) and extremely useful as a medicament.

The compound (I) or a prodrug thereof (hereinafter they are sometimes to be abbreviated collectively as compound (II)) shows low toxicity and can be used as an agent for the prophylaxis or treatment of various diseases to be mentioned later for mammals (e.g., humans, mice, rats, rabbits, dogs, cats, bovines, horses, pigs, monkeys) as it is or by admixing with a pharmacologically acceptable carrier and the like to give a pharmaceutical composition.

Here, various organic or inorganic carriers conventionally usable as materials for pharmaceutical preparations are used as a pharmacologically acceptable carrier, which are exemplified by excipient, lubricant, binder and disintegrant for solid preparations; solvent, solubilizing agent, suspending agent, isotonicity agent, buffer and soothing agent for liquid preparations, and the like. Where necessary, an additive for pharmaceutical preparations such as preservative, antioxidant, colorant, sweetening agent may be used for formulation.

Preferable examples of the excipient include lactose, sucrose, D-mannitol, D-sorbitol, starch, α-starch, dextrin, crystalline cellulose, low-substituted hydroxypropylcellulose, sodium carboxymethylcellulose, gum arabic, pullulan, light anhydrous silicic acid, synthetic aluminum silicate, and magnesium aluminate metasilicate.

Preferred examples of the lubricant include magnesium stearate, calcium stearate, talc, and colloidal silica.

Preferable examples of the binder include α-starch, saccharose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, and polyvinylpyrrolidone.

Preferable examples of the disintegrant include lactose, sucrose, starch, carboxymethylcellulose, calcium carboxymethylcellulose, sodium croscarmellose, sodium carboxymethylstarch, light anhydrous silicic acid, and low-substituted hydroxypropylcellulose.

Preferable examples of the solvent include water for injection, physiological brine, Ringer's solution, alcohol, propylene glycol, polyethylene glycol, sesame oil, corn oil, olive oil, and cottonseed oil.

Preferred examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate, and sodium acetate.

Preferred examples of the suspending agent include surfactants such as stearyltriethanolamine, sodium lauryl sulfate, lauryl aminopropionic acid, lecithin, benzalkonium chloride, benzethonium chloride, glyceryl monostearate; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose; polysorbates, and polyoxyethylene hydrogenated castor oil.

Preferred examples of the isotonicity agent include sodium chloride, glycerol, D-mannitol, D-sorbitol, and glucose.

Preferred examples of the buffer include buffers such as phosphate, acetate, carbonate and citrate.
Preferred examples of the soothing agent include benzyl alcohol.

Preferred examples of the preservative include p-oxybenzoates, chlorobutanol, benzyl alcohol, phenethyl alcohol, dehydroacetic acid, and sorbic acid.
Preferred examples of the antioxidant include sulfite, ascorbate, and the like.

Preferable examples of the colorant include water-soluble edible tar pigments (e.g., foodcolors such as Food Color Red Nos. 2 and 3, Food Color Yellow Nos. 4 and 5, Food Color Blue Nos. 1 and 2), water insoluble lake pigments (e.g., aluminum salt of the aforementioned water-soluble edible tar pigment), and natural pigments (e.g., β-carotene, chlorophil, ferric oxide red).

Preferable examples of the sweetening agent include saccharin sodium, dipotassium glycyrrhizinate, aspartame, and stevia.

The medicament containing compound (II) can be formulated by using compound (II) alone or by mixing the compound with a pharmacologically acceptable carrier according to a method known per se (e.g., the method described in the Japanese Pharmacopoeia etc.) as the production method of a pharmaceutical preparation, and can be safely administered in the form of, for example, tablet (including sugar-coated tablet, film-coated tablet, sublingual tablet, orally disintegrating tablet, buccal tablet and the like), pill, powder, granule, capsule (including soft capsule, microcapsule), troche, syrup, liquid, emulsion, suspension, release control preparation (e.g., quick-release preparation, sustained-release preparation, sustained-release microcapsule), aerosol, film (e.g., orally disintegrating film, oral mucosa-adhesive film), injection (e.g., subcutaneous injection, intravenous injection, intramuscular injection, intraperitoneal injection), drip infusion, transdermal absorption type preparation, ointment, lotion, adhesive preparation, suppository (e.g., rectal suppository, vaginal suppository), pellet, nasal preparation, pulmonary preparation (inhalant), eye drop and the like, orally or parenterally (e.g., intravenous, intramuscular, subcutaneous, intraorgan, intranasal, intradermal, instillation, intracerebral, intrarectal, intravaginal, intraperitoneal and intratumor administrations, administration to the vicinity of tumor, and direct administration to the lesion).

A pharmaceutical composition can be produced by a method conventionally used in the technical field of pharmaceutical preparation, for example, the method described in the Japanese Pharmacopoeia and the like.

While the content of compound (II) in the pharmaceutical composition varies depending on the dosage form, dose of compound (II), and the like, it is, for example, about 0.1 to 100 wt%.

During production of an oral preparation, coating may be applied as necessary for the purpose of masking of taste, enteric property or durability.

Examples of the coating base to be used for coating include sugar coating base, aqueous film coating base, enteric film coating base and sustained-release film coating base.

As the sugar coating base, sucrose is used. Moreover, one or more kinds selected from talc, precipitated calcium carbonate, gelatin, gum arabic, pullulan, carnauba wax and the like may be used in combination.

Examples of the aqueous film coating base include cellulose polymers such as hydroxypropyl cellulose, hydroxypropylmethyl cellulose, hydroxyethyl cellulose, methylhydroxyethyl cellulose; synthetic polymers such as polyvinylacetal diethylaminoacetate, aminoalkyl methacrylate copolymer E [Eudragit E (trade name)], polyvinylpyrrolidone; and polysaccharides such as pullulan.

Examples of the enteric film coating base include cellulose polymers such as hydroxypropylmethyl cellulose phthalate, hydroxypropylmethyl cellulose acetate succinate, carboxymethylethyl cellulose, cellulose acetate phthalate; acrylic polymers such as methacrylic acid copolymer L [Eudragit L (trade name)], methacrylic acid copolymer LD [Eudragit L-30D55 (trade name)], methacrylic acid copolymer S [Eudragit S (trade name)]; and naturally occurring substances such as shellac.

Examples of the sustained-release film coating base include cellulose polymers such as ethyl cellulose; and acrylic polymers such as aminoalkyl methacrylate copolymer RS [Eudragit RS (trade name)], ethyl acrylate-methyl methacrylate copolymer suspension [Eudragit NE (trade name)].

The above-mentioned coating bases may be used after mixing with two or more kinds thereof at appropriate ratios. For coating, for example, a light shielding agent such as titanium oxide, red ferric oxide can be used.

The compound (II) shows low toxicity (e.g., acute toxicity, chronic toxicity, genetic toxicity, reproductive toxicity, cardiotoxicity, carcinogenicity and the like) and a few side effects. Therefore, it can be used as an agent for the prophylaxis or treatment or a diagnostic of various diseases in a mammal (e.g., human, bovine, horse, dog, cat, monkey, mouse, rat).

Since compound (II) has a promoting activity for a muscle cell differentiation, it is useful as a prophylactic or therapeutic drug for the diseases developed by or whose development depends onthe suppression of muscle differentiation, or by a factor induced by the suppression of muscle differentiation.

Examples of the diseases developed by or whose development depends on the suppression of muscle differentiation, or by a factor induced by the suppression of muscle differentiation include diabetes, glucose tolerance disorders, ketosis, acidosis, diabetic complications [e.g., neuropathy, nephropathy, retinopathy, cataract, macroangiopathy, osteopenia, hyperosmolar diabetic coma, infections (e.g., respiratory infection, urinary tract infection, gastrointestinal infection, dermal soft tissue infections, inferior limb infection etc.), diabetic gangrene, xerostomia, hypacusis, cerebrovascular disorder, peripheral blood circulation disorder], obesity and obesity complications, sleep apnea syndrome, osteoporosis, cachexia (e.g., carcinomatous cachexia, tuberculous cachexia, diabetic cachexia, hemopathic cachexia, endocrinopathic cachexia, infectious cachexia or cachexia induced by acquired immunodeficiency syndrome etc.), fatty liver, nonalcoholic steatohepatitis, hypertension, polycystic ovary syndrome, renal disease (e.g., diabetic nephropathy, glomerulonephritis, glomerulosclerosis, nephrosis syndrome, hypertensive nephrosclerosis, end-stage renal disorder etc.), myogenic muscular atrophy (muscular dystrophy, myotonic dystrophy, myopathy etc.), neurogenic muscular atrophy (amyotrophic lateral sclerosis, spinobulbar muscular atrophy, spinal muscular atrophy, hereditary neuropathy etc.), disuse muscular atrophy, sarcopenia, myocardial infarction, angina pectoris, cardiac failure, cerebrovascular disorder (e.g., cerebral infarction, cerebral apoplexy etc.), insulin resistance syndrome, syndrome X, metabolic syndrome (pathology involving not less than three selected from hyper-triglycerid(TG)emia, low HDL cholesterol(HDL-C)emia, hypertension, abdomen obesity and impaired glucose tolerance), hyperinsulinemia, perception disorder in hyperinsulinemia, tumor (e.g., leukemia, breast cancer, prostate cancer, skin cancer etc.), irritable bowel syndrome, acute or chronic diarrhea, inflammatory disease (e.g., arteriosclerosis (e.g., atherosclerosis etc.), rheumatoid arthritis, spondylitis deformans, osteoarthritis, lumbago, gout, hyperuricemia, inflammation after operation or trauma, swelling, neuralgia, pharyngolaryngitis, cystitis, hepatitis (including nonalcoholic steatohepatitis), pneumonia, pancreatitis, inflammatory colitis, ulcerative colitis etc.), visceral obesity syndrome, macular edema, dyslipidemia, sexual dysfunction, muscular atrophy, dermatic diseases, arthropathy, osteopenia, arteriosclerosis, thrombotic disease, dyspepsia, deficits in memory and learning, depression, manic-depressive illness, schizophrenia, attention deficit hyperactivity disorder, vision disorder, appetite regulation disorder (e.g., hyperorexia etc.), hypoglycemia, edema, insulin resistance, unstable diabetes, fatty atrophy, insulin allergy, insulinoma, lipotoxicity, cancer (e.g., breast cancer etc.), immune diseases (e.g., immunodeficiency etc.), multiple sclerosis, acute renal failure and the like can be mentioned. Here, diabetes includes type 1 diabetes, type 2 diabetes, gestational diabetes and obese diabetes. Dyslipidemia includes hypertriglyceridemia, hypercholesterolemia, hypo HDL-emia, postprandial hyperlipidemia and the like.

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 h level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 h level (glucose concentration of intravenous plasma) of less than 140 mg/dl" (normal type) is called a "borderline type".

In addition, ADA (American Diabetes Association) and WHO reported diagnostic criteria of diabetes.

According to these reports, diabetes is a condition showing a fasting blood glucose level (glucose concentration of intravenous plasma) of not less than 126 mg/dl, or a 75 g oral glucose tolerance test 2 h level (glucose concentration of intravenous plasma) of not less than 200 mg/dl.

According to the above-mentioned reports by ADA and WHO, impaired glucose tolerance is a condition showing a 75 g oral glucose tolerance test 2 h level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). According to the report of WHO, among the IFG (Impaired Fasting Glucose), a condition showing a 75 g oral glucose tolerance test 2 h level (glucose concentration of intravenous plasma) of not less than 110 mg/dl and less than 126 mg/dl is called IFG (Impaired Fasting Glycemia).

The compound (II) can also be used as an agent for the prophylaxis or treatment of diabetes, borderline type, abnormal glucose tolerance, IFG (Impaired Fasting Glucose) and IFG (Impaired Fasting Glycemia), as determined according to the above-mentioned diagnostic criteria. Moreover, compound (II) can prevent progress of borderline type, abnormal glucose tolerance, IFG (Impaired Fasting Glucose) or IFG (Impaired Fasting Glycemia) into diabetes.

Since compound (II) has a suppressive activity for adipocyte differentiation, it is useful as a prophylactic or therapeutic drug for a disease developed by or whose development depends on promoted adipocyte differentiation, or by a factor induced by promoted adipocyte differentiation.
Examples of the disease which is developed by or whose development depends on promoted adipocyte differentiation, or by a factor induced by promoted adipocyte differentiation include obesity complications, sleep apnea syndrome, osteoporosis, cachexia (e.g., carcinomatous cachexia, tuberculous cachexia, diabetic cachexia, hemopathic cachexia, endocrinopathic cachexia, infectious cachexia or cachexia induced by acquired immunodeficiency syndrome etc.), fatty liver, hypertension, polycystic ovary syndrome, muscular dystrophy, cerebrovascular disorder (e.g., cerebral infarction, cerebral apoplexy etc.), metabolic syndrome (pathology having three or more selected from hyper-triglycerid(TG)emia, low HDL cholesterol(HDL-C) emia, hypertension, abdomen obesity and impaired glucose tolerance), hyperinsulinemia, perception disorder in hyperinsulinemia, irritable bowel syndrome, acute or chronic diarrhea, swelling, neuralgia, hepatitis (including nonalcoholic steatohepatitis), cardiac disease (e.g., cardiac hypertrophy, acute cardiac failure and chronic cardiac failure including congestive cardiac failure, cardiomyopathy, angina pectoris, myocarditis, arrhythmia, tachycardia, myocardial infarction etc.), cardiac muscle ischemia, venous insufficiency, progression to cardiac failure after myocardial infarction, hypertension, cor pulmonale, arteriosclerosis including atherosclerosis (e.g., aneurysm, coronary sclerosis, cerebral arteriosclerosis, peripheral arterial sclerosis etc.), vascular hypertrophy, intervention (e.g., percutaneous transluminal coronary angioplasty, stenting, coronary angioscopy, intravascular ultrasound, douche thrombolytic therapy etc.) and vascular hypertrophy or obstruction and organ disorder after heart transplantation, blood vessel reocclusion or restenosis after bypass surgery, respiratory diseases (e.g., cold syndrome, pneumonia, asthma, pulmonary hypertension, pulmonary thrombi or pulmonary obliteration etc.), bone disease (e.g., bone fracture, bone refracture, bone deformation or spondylosis deformans, osteosarcoma, myeloma, dysosteogenesis, non-metabolic bone disease such as lateral curvature, bone defect, osteoporosis, osteomalacia, rickets, osteitis fibrosis, renal osteodystrophy, Paget's disease of bone, rigid myelitis, rheumatoid arthritis, osteoarthrosis of knee and destruction of articular tissue in diseases similar thereto etc.), inflammatory disease (e.g., retinopathy, nephropathy, neuropathy, rheumatoid arthritis, osteoarthritis, rheumatoid myelitis, arthritis such as periostitis, inflammation after operation or trauma, remission of swelling, pharyngitis, cystitis, atopic dermatitis, inflammatory bowel disease such as Crohn's disease and ulcerative colitis, meningitis, inflammatory ocular disease, inflammatory pulmonary disease such as pneumonia, silicosis, lung sarcoidosis and pulmonary tuberculosis), allergic disease (e.g., allergic rhinitis, conjunctivitis, gastrointestinal allergy, pollinosis, anaphylaxis etc.), drug dependence, neurodegenerative disease (e.g., Alzheimer's disease, Parkinson's disease, amyotrophic lateral sclerosis, AIDS encephalopathy etc.), central nervous disorders (e.g., disorder such as cerebral hemorrhage and cerebral infarction and sequelae or complications thereof, head trauma, spinal injury, brain edema etc.), dementia, memory disorders, disturbance of consciousness, amnesia, anxiety symptom, catatonic symptom, discomfort mental state, mental diseases (e.g., depression, epilepsy, alcohol dependence etc.), ischemic peripheral circulation disorder, deep vein thrombosis, obstructive peripheral circulation disorder, arteriosclerosis obliterans, thromboangiitis obliterans, diabetes (e.g., type 1 diabetes, type 2 diabetes, type 1.5 diabetes (LADA(Latent Autoimmune Diabetes in Adults)), gestational diabetes, diabetes with impaired insulin secretion, obese diabetes, impaired glucose tolerance (IGT (Impaired Glucose Tolerance)), IFG (Impaired Fasting Glucose), IFG (Impaired Fasting Glycaemia) etc.), diabetic complications [e.g., neuropathy, nephropathy, retinopathy, cataract, macroangiopathy, osteopenia, hyperosmolar diabetic coma, infections (e.g., respiratory infection, urinary tract infection, gastrointestinal infection, dermal soft tissue infections, inferior limb infection etc.), diabetic gangrene, xerostomia, hypacusis, cerebrovascular disorder, peripheral blood circulation disorder etc.], incontinence, disorder of metabolism or nutrition (e.g., obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity etc.), hyperphagia etc.), hyperlipidemia, hypercholesterolemia, impaired glucose tolerance etc.), insulin resistance syndrome, syndrome X, visceral obesity syndrome, male or female sexual dysfunction, cerebrovascular disorder (e.g., asymptomatic cerebrovascular accident, transient cerebral ischemic attack, cerebral apoplexy, cerebrovascular dementia, hypertensive encephalopathia, cerebral infarction etc.), brain edema, brain circulation disorder, recurrence and sequelae of cerebrovascular disorder (e.g., neural symptoms, mental symptoms, subjective symptoms, disorders of daily living activities etc.), renal diseases (e.g., nephritis, glomerulonephritis, glomerulosclerosis, renal failure, thrombotic microangiopathy, diabetic nephropathy, nephrosis syndrome, hypertensive nephrosclerosis, dialysis complications, organ disorder including nephropathy due to radiation etc.), ophthalmic diseases (e.g., glaucoma, ocular hypertension disease etc.), thrombosis, multiple organ failure, endothelial dysfunction, and other circulatory diseases thereof (e.g., ischemic cerebral circulatory disorder, Raynaud's disease, Buerger's disease etc.), chronic obstructive pulmonary diseases, interstitial pneumonia, carinii pneumonia, collagen disease (e.g., systemic lupus erythematosus, scleroderma, polyarteritis etc.), liver disease (e.g., hepatitis including chronic one, cirrhosis etc.), digestive tract diseases (e.g., gastritis, gastric ulcer, gastric cancer, postgastrostomy disorder, dyspepsia, esophageal ulcer, pancreatitis, colon polyp, cholelithiasis, hemorrhoids, variceal ruptures of esophagus or stomach etc.), blood or hematopoietic organ disease (e.g., polycythemia, vascular peliosis, autoimmune hemolytic anemia, disseminated intravascular coagulation syndrome, multiple myelopathy etc.), solid tumor, tumor (e.g., malignant melanoma, malignant lymphoma, cancer of gastrointestinal organ (e.g., stomach, intestine etc.) etc.), cancer and cachexia following cancer, metastasis cancer, endocrine diseases (e.g., Addison's disease, Cushing's syndrome, melanocytoma, primary aldosteronism etc.), urinary organ or male genital disease (e.g., cystitis, prostatic hyperplasia, prostatic cancer, sexually-transmitted diseases etc.), gynecological diseases (e.g., climacteric disorder, gestational toxicosis, endometriosis, hysteromyoma, ovarian disease, mammary disease, sexually-transmitted diseases etc.), infections (e.g., virus infections such as cytomegalovirus, influenza virus, herpes virus, rickettsial infections, bacterium infections etc.), toxemia (e.g., sepsis, septic shock, endotoxin shock, gram negative sepsis, toxic shock syndrome etc.), dermatic diseases (e.g., keloid, hemangioma, psoriasis etc.) and the like. The compound (II) is desirably used particularly for the prophylaxis or treatment of diabetes, obesity, dyslipidemia and the like.

Furthermore, since compound (II) has an action to suppress NF-kB activation, it is useful as a prophylactic or therapeutic drug for the diseases developed by or whose development depends on NF-kB activation, or by a factor induced by NF-kB activation.

A disease developed by or whose development depensds on NF-kB activation, or by a factor induced by NF-kB activation includes various inflammatory diseases. Examples of such inflammatory disease include arthritis (e.g., rheumatoid arthritis, spondylitis deformans, osteoarthritis, lumbago, rheumatoid myelitis, gouty arthritis, synovitis etc.), asthma, pharyngolaryngitis, cystitis, hepatitis, pneumonia, allergic disease, arteriosclerosis including atherosclerosis (e.g., aneurysm, coronary sclerosis, cerebral arterial sclerosis, peripheral arterial sclerosis etc.), gastric mucosa injury (including gastric mucosa injury caused by aspirin), digestive tract diseases such as inflammatory bowel disease (e.g., Crohn's disease, ulcerative colitis etc.), diabetic complications (e.g., retinopathy, nephropathy, diabetic neuropathy, diabetic vascular disorder etc.), atopic dermatitis, chronic obstructive pulmonary diseases, systemic lupus erythematosus, visceral inflammatory disease (e.g., nephritis, hepatitis, nonalcoholic steatohepatitis etc.), autoimmune hemolytic anemia, psoriasis, neurodegenerative disease (e.g., Alzheimer's disease, Parkinson's disease, amyotrophic lateral sclerosis, AIDS encephalopathy etc.), central nervous disorders (e.g., cerebrovascular disorder such as cerebral hemorrhage and cerebral infarction, head trauma, spinal damage, brain edema, multiple sclerosis etc.), meningitis, myocarditis, cardiomyopathy, ischemic cardiac diseases, angina pectoris, myocardial infarction, cardiac failure, vascular hypertrophy or obstruction after intervention (e.g., percutaneous transluminal coronary angioplasty, stenting, congestive coronary angioscopy, intravascular ultrasound, intracoronary thrombolysis etc.) and organ disorder, reocclusion or restenosis after bypass operation, endothelial functional disorder, other circulatory diseases (e.g., intermittent claudication, obstructive peripheral circulation disorder, arteriosclerosis obliterans, thromboangiitis obliterans, ischemic cerebral circulatory disorder, Raynaud's disease, Buerger's disease etc.), inflammatory ocular disease, inflammatory pulmonary disease (e.g., chronic pneumonia, silicosis, lung sarcoidosis, pulmonary tuberculosis etc.), endometriosis, toxemia (e.g., sepsis, septic shock, endotoxin shock, gram negative sepsis, toxin shock syndrome etc.), cachexia (e.g., cachexia due to infection, carcinomatous cachexia, cachexia induced by acquired immunodeficiency syndrome etc.), cancer, Addison's disease, Creutzfeldt-Jakob disease, virus infection (e.g., virus infection such as cytomegalovirus, influenza virus, herpes virus etc.), disseminated intravascular coagulation and the like.

Since compound (II) has a suppressive activity for body weight gain, it can be used as a body weight gain suppressor to mammals. Target mammals may be any mammals of which body weight gain is to be avoided. The mammals may have a risk of body weight gain genetically or may be suffering from lifestyle-related diseases such as diabetes, hypertension and/or hyperlipemia. The body weight gain may be caused by excessive feeding or diet without nutrient balance, or may be derived from concomitant drug (e.g., agents for enhancing insulin sensitivity having PPARγ-agonistic activity such as troglitazone, rosiglitazone, englitazone, ciglitazone, pioglitazone etc.). In addition, body weight gain may be preliminary to obesity, or may be body weight gain of obesity patients. Here, obesity is defined that BMI (body mass index; body weight (kg)/[height (m)]²) is not less than 25 for Japanese (criterion by Japan Society for the Study of Obesity), or not less than 30 for westerner (criterion by WHO).

The compound (II) is also useful as an agent for the prophylaxis or treatment of metabolic syndrome. Because patients with metabolic syndrome have an extreme high incidence of cardiovascular diseases as compared to patients with single lifestyle-related disease, the prophylaxis or treatment of metabolic syndrome is quite important to prevent cardiovascular diseases.
Criteria for diagnosis of metabolic syndrome are announced by WHO in 1999, and by NCEP in 2001. According to the criterion of WHO, patients with at least two of abdominal obesity, dyslipidemia (high TG or low HDL) and hypertension in addition to hyperinsulinemia or impaired glucose tolerance are diagnosed as metabolic syndrome (World Health Organization: Definition, Diagnosis and Classification of Diabetes Mellitus and Its Complications. Part I: Diagnosis and Classification of Diabetes Mellitus, World Health Organization, Geneva, 1999). According to the criterion of Adult Treatment Panel III of National Cholesterol Education Program, that is an indicator for managing ischemic heart diseases in America, patients with at least three of abdominal obesity, high triglycerides, low HDL cholesterolemia, hypertension and impaired glucose tolerance are diagnosed as metabolic syndrome (National Cholesterol Education Program: Executive Summary of the Third Report of National Cholesterol Education Program (NCEP) Expert Panel on Detection, Evaluation, and Treatment of High Blood Cholesterol in Adults (Adults Treatment Panel III). The Journal of the American Medical Association, Vol. 285, 2486-2497, 2001).

The dose of compound (II) of the present invention can be appropriately selected according to the subject of administration, administration route, target disease, symptom and the like. For example, the dose of compound (II) for oral administration to an adult patient with diabetes, obesity, dyslipidemia, steatohepatitis, cachexia, or muscular atrophy is generally about 0.001 - 50 mg/kg body weight, preferably about 0.01 - 45 mg/kg body weight, more preferably about 0.1 - 2 mg/kg, as a single dose of the active ingredient "compound (I)". This amount is desirably administered 1-3 times per day.

Moreover, compound (II) can be used in combination with a drug other than compound (II).

Examples of the drug that can be used in combination with compound (II) (hereinafter sometimes to be abbreviated as concomitant drug) include a therapeutic agent for diabetes, a therapeutic agent for diabetic complications, a therapeutic agent for hyperlipemia, an antihypertensive agent, an antiobesity agent, a diuretic, a chemotherapeutic agent, an immunotherapeutic agent, an anti-inflammatory drug, an antithrombotic agent, a therapeutic agent for osteoporosis, a vitamin drug, an antidementia agent, a therapeutic agent for pollakiuria or urinary incontinence, a therapeutic agent for dysuria and the like.

A combination drug for compound (II) specifically includes the following.

Examples of another therapeutic agents for diabetes include insulin preparations (e.g., animal insulin preparations extracted from pancreas of bovine or swine; human insulin preparations genetically synthesized using *Escherichia coli,* yeast; zinc insulin; protamine zinc insulin; fragment or derivative of insulin (e.g., INS-1 etc.), oral insulin preparation etc.), insulin sensitizers (e.g., pioglitazone or a salt thereof (preferably hydrochloride), rosiglitazone or a salt thereof (preferably maleate), Metaglidasen, AMG-131, Balaglitazone, MBX-2044, Rivoglitazone, Aleglitazar, Chiglitazar, Lobeglitazone, PLX-204, PN-2034, GFT-505, THR-0921, compound described in WO 2007/013694, WO 2007/018314, WO 2008/093639 or WO 2008/099794), α-glucosidase inhibitors (e.g., voglibose, acarbose, miglitol, emiglitate etc.), biguanides (e.g., metformin, buformin or a salt thereof (e.g., hydrochloride, fumarate, succinate etc.) etc.), insulin secretagogues [e.g. sulfonylureas (e.g., tolbutamide, glibenclamide, gliclazide, chlorpropamide, tolazamide, acetohexamide, glyclopyramide, glimepiride, glipizide, glybuzole etc.), repaglinide, nateglinide, mitiglinide or a calcium salt hydrate thereof etc.], dipeptidyl peptidase IV inhibitors (e.g., Alogliptin or a salt thereof (preferably benzoate), Vildagliptin, Sitagliptin, Saxagliptin, BI1356, GRC8200, MP-513, PF-00734200, PHX1149, SK-0403, ALS2-0426, TA-6666, TS-021, KRP-104, 2-[[6-[(3R)-3-amino-1-piperidynyl]-3,4-dihydro-3-methyl-2,4-dioxo-1(2H)-pyrimidinyl]methyl]-4-fluorobenzonitrile or a salt thereof etc.), β3 agonists (e.g., N-5984 etc.), GPR40 agonists (e.g., compound described in WO 2004/041266, WO 2004/106276, WO 2005/063729, WO 2005/063725, WO 2005/087710, WO 2005/095338, WO 2007/013689 or WO 2008/001931 etc.), GLP-1 receptor agonists [e.g., GLP-1, GLP-1MR preparation, Liraglutide, Exenatide, AVE-0010, BIM-51077, Aib(8,35)hGLP-1(7,37)NH2, CJC-1131, Albiglutide etc.], amylin agonists (e.g., pramlintide etc.), phosphotyrosine phosphatase inhibitors (e.g., sodium vanadate etc.), gluconeogenesis inhibitors (e.g., glycogen phosphorylase inhibitors, glucose-6-phosphatase inhibitors, glucagon antagonists, FBPase inhibitors etc.), SGLT2 (sodium-glucose cotransporter 2) inhibitors (e.g., Depagliflozin, AVE2268, TS-033, YM543, TA-7284, Remogliflozin, ASP1941 etc.), SGLT1 inhibitors, 11β-hydroxysteroid dehydrogenase inhibitors (e.g., BVT-3498, INCB-13739 etc.), adiponectin or an agonist thereof, IKK inhibitors (e.g., AS-2868 etc.), leptin resistance improving drugs, somatostatin receptor agonists, glucokinase activators (e.g., Piragliatin, AZD1656, AZD6370, AZD6714, TPP355, compound described in WO 2006/112549, WO 2007/028135, WO 2008/047821, WO 2008/050821, WO 2008/136428 or W02008/156757 etc.), GIP (Glucose-dependent insulinotropic peptide), GPR119 agonists (e.g., PSN821, MBX-2982, APD597 etc.), FGF21, FGF analogs and the like.

Examples of the therapeutic agents for diabetic complications include aldose reductase inhibitors (e.g., tolrestat, epalrestat, zopolrestat, fidarestat, CT-112, ranirestat (AS-3201), lidorestat etc.), neurotrophic factor and increasing drugs thereof (e.g., NGF, NT-3, BDNF, neurotrophin production/secretion promoting agent (e.g., 4-(4-chlorophenyl)-2-(2-methyl-1-imidazolyl)-5-[3-(2-methylphenoxy)propyl]oxazole etc.) described in WO 01/14372, a compound described in WO 2004/039365, etc.), PKC inhibitors (e.g., ruboxistaurin mesylate etc.), AGE inhibitors (e.g., ALT946, N-phenacylthiazolium bromide (ALT766), EXO-226, Pyridorin, pyridoxamine etc.), GABA receptor agonists (e.g., gabapentin, Pregabalin etc.), serotonin noradrenaline re-uptake inhibitors (e.g., duloxetine etc.), sodium channel inhibitors (e.g., lacosamide etc.), active oxygen scavengers (e.g., thioctic acid etc.), cerebral vasodilators (e.g., tiapuride, mexiletine etc.), somatostatin receptor agonists (e.g., BIM23190 etc.), apoptosis signal regulating kinase-1 (ASK-1) inhibitors and the like.

Examples of the therapeutic agent for hyperlipemia include HMG-CoA reductase inhibitors (e.g., pravastatin, simvastatin, lovastatin, atorvastatin, fluvastatin, rosuvastatin, pitavastatin or a salt thereof (e.g., sodium salt, calcium salt etc.) etc.), squalene synthase inhibitors (e.g., a compound described in WO 97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid etc.), fibrate compounds (e.g., bezafibrate, clofibrate, simfibrate, clinofibrate etc.), anion exchange resins (e.g., colestyramine etc.), probucol, nicotinic acid drugs (e.g., nicomol, niceritrol, niaspan etc.), ethyl icosapentate, phytosterols (e.g., soysterol, γ-oryzanol etc.), cholesterol absorption inhibitors (e.g., Zetia etc.), CETP inhibitors (e.g., dalcetrapib, anacetrapib etc.), ω-3 fatty acid preparations (e.g., ω-3-acid ethyl esters 90 etc.) and the like.

Examples of the antihypertensive agent include angiotensin converting enzyme inhibitors (e.g., captopril, enalapril, delapril etc.), angiotensin II antagonists (e.g., candesartan cilexetil, candesartan, losartan, losartan potassium, eprosartan, valsartan, telmisartan, irbesartan, tasosartan, olmesartan, olmesartan medoxomil, azilsartan, azilsartan medoxomil etc.), calcium antagonists (e.g., manidipine, nifedipine, amlodipine, efonidipine, nicardipine, amlodipine, cilnidipine etc.), β blockers (e.g., metoprolol, atenolol, propranolol, carvedilol, pindolol etc.), clonidine and the like.

Examples of the antiobesity agent include monoamine uptake inhibitors (e.g., phentermine, sibutramine, mazindol, fluoxetine, tesofensine etc.), serotonin 2C receptor agonists (e.g., lorcaserin etc.), serotonin 6 receptor antagonists, histamine H3 receptor GABA modulators (e.g., topiramate etc.), neuropeptide Y antagonists (e.g., velneperit etc.), cannabinoid receptor antagonists (e.g., rimonabant, taranabant etc.), ghrelin antagonists, ghrelin receptor antagonists, ghrelinacylation enzyme inhibitors, opioid receptor antagonists (e.g., GSK-1521498 etc.), orexin receptor antagonists, melanocortin 4 receptor agonists, 11β-hydroxysteroid dehydrogenase inhibitors (e.g., AZD-4017 etc.), pancreatic lipase inhibitors (e.g., orlistat, cetilistat etc.), β3 agonists (e.g., N-5984 etc.), diacylglycerol acyltransferase 1 (DGAT1) inhibitors, acetylCoA carboxylase (ACC) inhibitors, stearoyl-CoA desaturated enzyme inhibitors, microsomal triglyceride transfer protein inhibitors (e.g., R-256918 etc.), Na-glucose cotransporter inhibitors (e.g., JNJ-28431754, remogliflozin etc.), NFκ inhibitors (e.g., HE-3286 etc.), PPAR agonists (e.g., GFT-505, DRF-11605 etc.), phosphotyrosine phosphatase inhibitors (e.g., sodium vanadate, Trodusquemin etc.), GPR119 agonists (e.g., PSN-821 etc.), glucokinase activators (e.g., AZD-1656 etc.), leptin, leptin derivatives (e.g., metreleptin etc.), CNTF (ciliary neurotrophic factor), BDNF (brain-derived neurotrophic factor), cholecystokinin agonists, glucagon-like peptide-1 (GLP-1) preparations (e.g., animal GLP-1 preparations extracted from the pancreas of bovine and pig; human GLP-1 preparations genetically synthesized using *Escherichia coli* or yeast; fragments or derivatives of GLP-1 (e.g., exenatide, liraglutide etc.) etc.), amylin preparations (e.g., pramlintide, AC-2307 etc.), neuropeptide Y agonists (e.g., PYY3-36, derivatives of PYY3-36, obineptide, TM-30339, TM-30335 etc.), oxyntomodulin preparations: FGF21 preparations (e.g., animal FGF21 preparations extracted from the pancreas of bovine and pig; human FGF21 preparations genetically synthesized using *Escherichia coli* or yeast; fragments or derivatives of FGF21 etc.), anorexigenic agents (e.g., P-57 etc.) and the like.

Examples of the diuretics include xanthine derivatives (e.g., theobromine sodium salicylate, theobromine calcium salicylate etc.), thiazide preparations (e.g., ethiazide, cyclopenthiazide, trichloromethiazide, hydrochlorothiazide, hydroflumethiazide, benzylhydrochlorothiazide, penflutizide, poly5thiazide, methyclothiazide etc.), antialdosterone preparations (e.g., spironolactone, triamterene etc.), carbonic anhydrase inhibitors (e.g., acetazolamide etc.), chlorobenzenesulfonamide agents (e.g., chlortalidone, mefruside, indapamide etc.), azosemide, isosorbide, ethacrynic acid, piretanide, bumetanide, furosemide and the like.

Examples of the chemotherapeutic agents include alkylating agents (e.g., cyclophosphamide, ifosfamide etc.), metabolic antagonists (e.g., methotrexate, 5-fluorouracil etc.), antitumor antibiotics (e.g., mitomycin, adriamycin etc.), plant-derived antitumor agents (e.g., vincristine, vindesine, Taxol etc.), cisplatin, carboplatin, etoposide and the like. Of these, Furtulon or NeoFurtulon, which are 5-fluorouracil derivatives, and the like are preferable.

Examples of the immunotherapeutic agents include microorganism or bacterial components (e.g., muramyl dipeptide derivatives, Picibanil etc.), polysaccharides having immunity potentiating activity (e.g., lentinan, schizophyllan, krestin etc.), cytokines obtained by genetic engineering techniques (e.g., interferon, interleukin (IL) etc.), colony stimulating factors (e.g., granulocyte colony stimulating factor, erythropoietin etc.) and the like, with preference given to interleukins such as IL-1, IL-2, IL-12.

Examples of the anti-inflammatory drug include non-steroidal anti-inflammatory drugs such as aspirin, acetaminophen, indomethacin and the like.

Examples of the antithrombotic agent include heparins (e.g., heparin sodium, heparin calcium, enoxaparin sodium, dalteparin sodium etc.), warfarins (e.g., warfarin potassium etc.), anti-thrombin drugs (e.g., argatroban, dabigatran etc.), FXa inhibitors (e.g., rivaroxaban, apixaban, edoxaban, YM150, compound described in WO 02/06234, WO 2004/048363, WO 2005/030740, WO 2005/058823 or WO 2005/113504 etc.), thrombolytic agents (e.g., urokinase, tisokinase, alteplase, nateplase, monteplase, pamiteplase etc.), platelet aggregation inhibitors (e.g., ticlopidine hydrochloride, clopidogrel, prasugrel, E5555, SHC530348, cilostazol, ethyl icosapentate, beraprost sodium, sarpogrelate hydrochloride etc.) and the like.

Examples of the therapeutic agents for osteoporosis include alfacalcidol, calcitriol, elcatonin, calcitonin salmon, estriol, ipriflavone, pamidronate disodium, alendronate sodium hydrate, incadronate disodium, risedronate disodium and the like.

Examples of the vitamins include vitamin B₁, vitamin B₁₂ and the like.

Examples of the antidementia agents include tacrine, donepezil, rivastigmine, galanthamine and the like.

Examples of the therapeutic agents for pollakiuria or urinary incontinence include flavoxate hydrochloride, oxybutynin hydrochloride, propiverine hydrochloride and the like.

Examples of the therapeutic agents for dysuria include acetylcholine esterase inhibitors (e.g., distigmine etc.) and the like.

Furthermore, drugs having a cachexia-improving action established in animal models and clinical situations, such as cyclooxygenase inhibitors (e.g., indomethacin etc.), progesterone derivatives (e.g., megestrol acetate etc.), glucosteroids (e.g., dexamethasone etc.), metoclopramide agents, tetrahydrocannabinol agents, fat metabolism improving agents (e.g., eicosapentanoic acid etc.), growth hormones, IGF-1, or antibodies to a cachexia-inducing factor such as TNF-α, LIF, IL-6, oncostatin M, can be used in combination with compound (II).

Furthermore, glycosylation inhibitors (e.g., ALT-711 etc.), nerve regeneration promoter (e.g., γ-128, VX853, prosaptide etc.), antidepressant (e.g., desipramine, amitriptyline, imipramine etc.), antiepileptic drugs (e.g., lamotrigine, Trileptal, Keppra, Zonegran, Pregabalin, Harkoseride, carbamazepine etc.), antiarrhythmic (e.g., mexiletine etc.), acetylcholine receptor ligand (e.g., ABT-594 etc.), endothelin receptor antagonist (e.g., ABT-627 etc.), monoamine uptake inhibitors (e.g., tramadol etc.), narcotic analgesics (e.g., morphine etc.), GABA receptor agonist (e.g., gabapentin, gabapentin MR agent etc.), α2 receptor agonist (e.g., clonidine etc.), topical analgesic (e.g., capsaicin etc.), antianxiety drug (e.g., benzothiazepine etc.), phosphodiesterase inhibitor (e.g., sildenafil etc.), dopamine receptor agonist (e.g., apomorphine etc.), midazolam, ketoconazole and the like can also be used in combination with compound (II).

The administration time of compound (II) and the concomitant drug is not restricted, and these can be administered to an administration subject simultaneously, or may be administered in a staggered manner.
The administration mode is not particularly restricted as long as compound (II) and the concomitant drug are combined. Examples of such administration mode include:
(1) administration of a single preparation obtained by simultaneously processing compound (II) and the concomitant drug,
(2) simultaneous administration of two kinds of preparations of compound (II) and the concomitant drug, which have been separately produced, by the same administration route,
(3) administration of two kinds of preparations of compound (II) and the concomitant drug, which have been separately produced, by the same administration route in a staggered manner,
(4) simultaneous administration of two kinds of preparations of compound (II) and the concomitant drug, which have been separately produced, by different administration routes,
(5) administration of two kinds of preparations of compound (II) and the concomitant drug, which have been separately produced, by different administration routes in a staggered manner (e.g., administration in the order of compound (II) and the concomitant drug, or in the reverse order) and the like.

The dose of the combination drug can be determined as appropriate based on the dose clinically employed. The proportion of compound (II) and the concomitant drug can be appropriately determined depending on the administration subject, administration route, target disease, condition, combination and the like. When, for example, the administration subject is human, the concomitant drug is used in an amount of 0.01-100 parts by weight per 1 part by weight of compound (II).

By combining compound (II) and a concomitant drug, a superior effect such as
(1) the dose can be reduced as compared to single administration of compound (II) or a concomitant drug,
(2) the period of treatment can be set longer by selecting a concomitant drug having different action and mechanism from compound (II),
(3) a sustained treatment effect can be designed by selecting a concomitant drug having different action and mechanism from compound (II),
(4) a synergistic effect can be afforded by a combined use of compound (II) and a concomitant drug, and the like, can be achieved.

The present invention is explained in more detail in the following by referring to Reference Examples and Examples, which are not to be construed as limitative.

In the following Reference Examples and Examples, the "room temperature" generally shows about 10°C to about 35°C.
The solvent used for chromatography is in % by volume, and others are in wt%.
In proton NMR spectrum, those that cannot be confirmed by broad such as OH and NH proton are not described in the data.
SD shows standard deviation.

Other abbreviations used herein mean the following.
s: singlet
d: doublet
t: triplet
q: quartet
m: multiplet
br: broad
J: coupling constant
Hz: hertz
CDCl₃: deuterated chloroform
DMSO-d₆: deuterated dimethyl sulfoxide
¹H NMR: proton nuclear magnetic resonance

In the following Reference Examples, the melting point, nuclear magnetic resonance spectrum (NMR) and mass spectrum (MS) were measured under the following conditions.

melting point measurement device: Yanagimoto micro melting point measurement device or Buchi melting point measurement device B-545 type.

NMR measurement device: Varian, Varian Gemini 300 (300MHz), Bruker BioSpin AVANCE 300.

MS measurement device: Waters ZQ2000, column: SHISEIDO CAPCELLPAK C18 UG120 1.5 mm I.D.x35 mm S-3 µm, solvent: solution A 0.05% trifluoroacetic acid-containing water, solution B 0.04% trifluoroacetic acid-containing acetonitrile, gradient cycle: 0.00 min (solution A/solution B = 90/10), 0.01 min (solution A/solution B = 90/10), 2.00 min (solution A/solution B = 5/95), 2.75 min (solution A/solution B = 5/95), 2.76 min (solution A/solution B = 90/10), 3.45 min (solution A/solution B = 90/10), flow rate: 0.5 mL/min, detection method: UV 220 nm, ionization method: ESI positive. Alternatively, MS measurement device: Agilent G6100, column: ZORBAX Extend-C18 Rapid Resolution HT 3.0×30 mm 1.8 micron 600 bar, solvent: solution A 0.01 M ammonium acetate-containing water, solution B 0.01 M ammonium acetate-containing acetonitrile, gradient cycle: 0.00 min (solution A/solution B = 90/10), 0.20 min (solution A/solution B = 90/10), 1.50 min (solution A/solution B = 0/100), 2.00 min (solution A/solution B = 90/10), flow rate: 1.2 mL/min, detection method: UV 220 nm, ionization method: ESI positive or ESI negative. The data described show actual data. Generally, a molecular ion peak is observed; however, when the compound has a tert-butoxycarbonyl group (-Boc), a peak of dissociated tert-butoxycarbonyl group or tert-butyl group may be observed as a fragment ion. When the compound has a hydroxyl group (-OH), a peak of dissociated H₂O may be observed as a fragment ion. In the case of a salt, generally, a molecular ion peak or fragment ion peak of a free form is observed.

Purification by preparative HPLC in the Reference Examples was performed under the following conditions. instrument: Waters preparative HPLC system, column: SunFire Prep C18 OBD5 µm 30×50 mm Column, solvent: solution A 0.1% trifluoroacetic acid-containing water, solution B 0.1% trifluoroacetic acid-containing acetonitrile, gradient cycle: 0.00 min (solution A/solution B = 90/10), 1.20 min (solution A/solution B = 90/10), 5.20 min (solution A/solution B = 0/100), 7.00 min (solution A/solution B = 0/100), 7.01 min (solution A/solution B = 90/10), 8.50 min (solution A/solution B = 90/10), flow rate: 70 mL/min, detection method: UV 220 nm. Alternatively, instrument: Waters preparative HPLC system, column: YMC CombiPrep ODS-A (50×20 mm. I.D S-5 µm, 12 nm), solvent: solution A 0.1% trifluoroacetic acid-containing water, solution B 0.1% trifluoroacetic acid-containing acetonitrile, gradient cycle: 0.00 min (solution A/solution B = 90/10), 0.20 min (solution A/solution B = 90/10), 4.20 min (solution A/solution B = 0/100), 6.30 min (solution A/solution B = 0/100), 6.31 min (solution A/solution B = 90/10), flow rate: 25 mL/min, detection method: UV 220 nm.

The compounds of Reference Examples 7 - 42 are disclosed in WO01/7482

### Examples

### Reference Example 1

### 1-methyl-8-[(2-methylpyridin-3-yl)oxy]-4,5-dihydro-1H-thieno[3,4-g]indazole-6-carboxamide

### a) 1-methyl-8-[(2-methylpyridin-3-yl)oxy]-4,5-dihydro-1H-thieno[3,4-g]indazole-6-carboxylic acid

To a solution of 3-hydroxy-2-methylpyridine (5.64 g) in N-methylpyrrolidone (160 ml) was added tert-butoxy potassium (6.3 g) and the mixture was stirred at room temperature for 1 hr. To this mixture was added 4,5-dihydro-1-methyl-8-methylsulfonyl-1H-thieno[3,4-g]indazole-6-carboxylic acid ethyl ester (16 g) and the mixture was stirred at 80°C for 2 hr. The reaction mixture was cooled, and diluted with water (480 ml). The mixture was extracted with ethyl acetate, and the extract was washed with water and 10% brine, and dried over anhydrous magnesium sulfate. The solvent was evaporated under reduced pressure to give 1-methyl-8-[(2-methylpyridin-3-yl)oxy]-4,5-dihydro-1H-thieno[3,4-g]indazole-6-carboxylic acid ethyl ester. The obtained 1-methyl-8-[(2-methylpyridin-3-yl)oxy]-4,5-dihydro-1H-thieno[3,4-g]indazole-6-carboxylic acid ethyl ester was dissolved in a mixture of ethanol (60 ml) and THF (60 ml). 1N Aqueous sodium hydroxide solution (60 ml) was added and the mixture was stirred at room temperature overnight. The reaction mixture was acidified with hydrochloric acid and the resulting precipitate was collected by filtration reduced pressure, washed with water and further dried to give the title compound (11.64 g).
¹H NMR (200 MHz, DMSO-d₆) δ2.50(3H, s), 2.65(2H, t, J=7.0 Hz), 3.17(2H, t, J=7.0 Hz), 4.00(3H, s), 7.36(1H, dd, J=8.4, 4.4 Hz), 7.38(1H, s), 7.77(1H, d, J=8.4Hz), 8.42(1H, d, J=4.4 Hz).

### b) 1-methyl-8-[(2-methylpyridin-3-yl)oxy]-4,5-dihydro-1H-thieno[3,4-g]indazole-6-carboxamide

1-Methyl-8-[(2-methylpyridin-3-yl)oxy]-4,5-dihydro-1H-thieno[3,4-g]indazole-6-carboxylic acid (3.4 g) was suspended in DMF (30 ml). To this solution were added WSC (2.3 g) and ammonia-HOBt complex (1.8 g), and the mixture was stirred at room temperature for 1 hr, diluted with water and extracted with ethyl acetate. The extract was washed with saturated brine, and dried over anhydrous magnesium sulfate. The solvent was evaporated under reduced pressure. The residue was stirred with ethanol (15 ml) for 1 hr and crystals were collected by filtration to give the title compound (1.44 g).
¹H NMR (200 MHz, DMSO-d₆) δ2.51(3H, s), 2.63(2H, t, J=6.8 Hz), 3.08 (2H, t, J=6.8 Hz), 3.98(3H, s), 7.35(1H, dd, J=8.2, 4.8 Hz), 7.36(1H, s), 7.44(2H, brs), 7.70(1H, dd, J=8.2, 1.2 Hz), 8.38(1H, dd, J=4.8, 1.2 Hz).

### Reference Example 2

### 8-(4-fluorophenoxy)-1-methyl-4,5-dihydro-1H-thieno[3,4-g]indazole-6-carboxamide

### a) 8-(4-fluorophenoxy)-1-methyl-4,5-dihydro-1H-thieno[3,4-g]indazole-6-carboxylic acid

To a solution of 4-fluorophenol (4.53 g) in N-methylpyrrolidone (120 ml) was added tert-butoxy potassium (4.8 g) and the mixture was stirred at room temperature for 1 hr. To this mixture was added 4,5-dihydro-1-methyl-8-methylsulfonyl-1H-thieno[3,4-g]indazole-6-carboxylic acid ethyl ester (12.3 g) and the mixture was stirred at 80°C for 2 hr. The reaction solution was cooled, diluted with water (360 ml) and extracted with ethyl acetate. The extract was washed with water and 10% brine, and dried over anhydrous magnesium sulfate. The solvent was evaporated under reduced pressure to give ethyl 8-(4-fluorophenoxy)-1-methyl-4,5-dihydro-1H-thieno[3,4-g]indazole-6-carboxylate. The obtained ethyl 8-(4-fluorophenoxy)-1-methyl-4,5-dihydro-1H-thieno[3,4-g]indazole-6-carboxylate was dissolved in a mixture of ethanol (50 ml) and THF(50 ml). 1N Aqueous sodium hydroxide solution (50 ml) was added and the mixture was stirred at room temperature overnight. The reaction solution was acidified with hydrochloric acid and the resulting precipitate was collected by filtration under reduced pressure, washed with water, and further dried to give the title compound (10.17 g).
¹H NMR (200 MHz, DMSO-d₆) δ2.63(2H, t, J=7.2 Hz), 3.16(2H, t, J=7.2 Hz), 3.98(3H, s), 7.28-7.54(5H, m).

### b) 8-(4-fluorophenoxy)-1-methyl-4,5-dihydro-1H-thieno[3,4-g]indazole-6-carboxamide

8-(4-Fluorophenoxy)-1-methyl-4,5-dihydro-1H-thieno[3,4-g]indazole-6-carboxylic acid (3.4 g) was suspended in DMF (30 ml). To this solution were added WSC (2.3 g) and ammonia-HOBt complex (1.8 g), and the mixture was stirred at room temperature for 1 hr, diluted with water and extracted with ethyl acetate. The extract was washed with saturated brine, and dried over anhydrous magnesium sulfate. The solvent was evaporated under reduced pressure. The residue was stirred with ethanol (10 ml) at 80°C for 1 hr and crystals were collected by filtration to give the title compound (2.28 g).
¹H NMR (200 MHz, DMSO-d₆) δ2.61(2H, t, J=7.4 Hz), 3.07(2H, t, J=7.4 Hz), 3.95(3H, s), 7.24-7.42(4H, m), 7.35(1H, s), 7.44(2H, brs).

### Reference Example 3

### 1-methyl-8-propoxy-4,5-dihydro-1H-thieno[3,4-g]indazole-6-carboxamide

### a) 1-methyl-8-propoxy-4,5-dihydro-1H-thieno[3,4-g]indazole-6-carboxylic acid

To a solution of 4,5-dihydro-1-methyl-8-methylsulfonyl-1H-thieno[3,4-g]indazole-6-carboxylic acid ethyl ester (1.0 g) and 1-propanol (0.4 g) in THF (20 mL) was added 60% sodium hydride (0.15 g) under ice-cooling. The reaction mixture was stirred under ice-cooling for 1 hr and further at room temperature for 40 min. 2N Hydrochloric acid was added and the mixture was extracted with ethyl acetate. The extract was washed with saturated brine, and dried over anhydrous magnesium sulfate. The solvent was evaporated under reduced pressure. The residue was purified by silica gel column chromatography(ethyl acetate/hexane) to give a mixture (0.55 g) of ethyl 1-methyl-8-propoxy-4,5-dihydro-1H-thieno[3,4-g]indazole-6-carboxylate and propyl 1-methyl-8-propoxy-4,5-dihydro-1H-thieno[3,4-g]indazole-6-carboxylate. This mixture was dissolved in a mixture of methanol (5 ml) and THF (15 ml). To the solution was added a solution of potassium hydroxide (0.25 g) in water (10 ml), and the mixture was stirred at room temperature for 12 hr. 2N Hydrochloric acid was added and the mixture was extracted with a mixed solvent of ethyl acetate-THF. The extract was washed with saturated brine, and dried over anhydrous magnesium sulfate. The solvent was evaporated under reduced pressure. The residue crystallized from THF-ethyl acetate to give the title compound (0.22 g).
¹H NMR (300 MHz, DMSO-d₆) δ1.01(3H, t, J=7.2 Hz), 1.86(2H, sext, J=7.2 Hz), 2.55(2H, t, J=6.6 Hz), 3.12(2H, t, J=6.6 Hz), 4.00(3H, s), 4.22(2H, t, J=7.0 Hz), 7.30(1H, s).

### b) 1-methyl-8-propoxy-4,5-dihydro-1H-thieno[3,4-g]indazole-6-carboxamide

To a solution of 1-methyl-8-propoxy-4,5-dihydro-1H-thieno[3,4-g]indazole-6-carboxylic acid (0.198 g) and ammonia-HOBt complex (0.127 g) in DMF (6 ml) was added WSC (0.16 g), and the mixture was stirred at room temperature for 16 hr. The reaction solution was poured into water, and the mixture was extracted with a mixed solvent of ethyl acetate-THF. The extract was washed with saturated brine, and dried over anhydrous magnesium sulfate. The solvent was evaporated under reduced pressure. The residue was crystallized from THF-IPE to give the title compound (0.14 g).
¹H NMR (300 MHz, DMSO-d₆) δ1.00(3H, t, J=7.0 Hz), 1.84(2H, sext, J=7.0 Hz), 2.53(2H, t, J=6.6 Hz), 3.00(2H, t, J=6.6 Hz), 3.99(3H, s), 4.17(2H, t, J=7.0 Hz), 7.29(1H, s), 7.33(2H, s).

### Reference Example 4

### 8-(2-methoxyethoxy)-1-methyl-4,5-dihydro-1H-thieno[3,4-g]indazole-6-carboxamide

### a) ethyl 8-(2-methoxyethoxy)-1-methyl-4,5-dihydro-1H-thieno[3,4-g] indazole-6-carboxylate

To a solution of 4,5-dihydro-1-methyl-8-methylsulfonyl-1H-thieno[3,4-g]indazole-6-carboxylic acid ethyl ester (2.02 g) and 2-methoxyethanol (0.7 ml) in THF (30 mL) was added 60% sodium hydride (0.30 g) under ice-cooling. The reaction mixture was stirred at room temperature for 2 hr, water was added, and the solvent was evaporated under reduced pressure. The residue was extracted with ethyl acetate, and the extract was washed with saturated brine, and dried over anhydrous magnesium sulfate. The solvent was evaporated under reduced pressure. The residue was purified by silica gel column chromatography (ethyl acetate/hexane) to give the title compound (0.82 g).
¹H NMR (300 MHz, DMSO-d₆) δ1.37(3H, t, J=7.4 Hz), 2.64(2H, t, J=6.6 Hz), 3.21(2H, t, J=6.6 Hz), 3.41(3H, s), 3.76-3.80(2H, m), 4.11(3H, s), 4.32(2H, q, J=7. 4Hz), 4.31-4.36(2H, m), 7.33(1H, s).

### b) 8-(2-methoxyethoxy)-1-methyl-4,5-dihydro-1H-thieno[3,4-g]indazole-6-carboxylic acid

A mixed solution of an aqueous solution of ethyl 8-(2-methoxyethoxy)-1-methyl-4,5-dihydro-1H-thieno[3,4-g]indazole-6-carboxylate (0.70 g), and potassium hydroxide (0.40 g) in water (20 ml) and ethanol (20 ml) was stirred at room temperature for 14 hr. 2N Hydrochloric acid was added and the mixture was extracted with a mixed solvent of ethyl acetate-THF. The extract was washed with saturated brine, and dried over anhydrous magnesium sulfate. The solvent was evaporated under reduced pressure to give the title compound (0.39 g).
¹H NMR (300 MHz, DMSO-d₆) δ2.56(2H, t, J=7.0 Hz), 3.11(2H, t, J=7.0 Hz), 3.32(3H, s), 3.74(2H, t, J=4.8 Hz), 3.99(3H, s), 4.37(2H, t, J=4.8 Hz), 7.30(1H, s).

### c) 8-(2-methoxyethoxy)-1-methyl-4,5-dihydro-1H-thieno[3,4-g]indazole-6-carboxamide

To a solution of 8-(2-methoxyethoxy)-1-methyl-4,5-dihydro-1H-thieno[3,4-g]indazole-6-carboxylic acid (0.31 g) and ammonia-HOBt complex (0.17 g) in DMF (10 ml) was added WSC (0.22 g), and the mixture was stirred at room temperature for 15 hr. The solvent was evaporated under reduced pressure. The residue was extracted with ethyl acetate, and the extract was washed with saturated aqueous sodium hydrogen carbonate solution and saturated brine, and dried over anhydrous magnesium sulfate. The solvent was evaporated under reduced pressure. The residue crystallized from ethyl acetate-hexane to give the title compound (0.22 g).
¹H NMR (300 MHz, DMSO-d₆) δ2.54(2H, t, J=6.6 Hz), 3.00(2H, t, J=6.6 Hz), 3.73(2H, t, J=4.4 Hz), 3.98(3H, s), 4.31(2H, t, J=4.4 Hz), 7.29(1H, s), 7.34(2H, s).

### Reference Example 5

### 1-methyl-8-(pyridin-4-ylmethoxy)-4,5-dihydro-1H-thieno[3,4-g]indazole-6-carboxamide

To a solution of 4,5-dihydro-1-methyl-8-methylsulfonyl-1H-thieno[3,4-g]indazole-6-carboxylic acid ethyl ester (1.50 g) and 4-pyridinemethanol (0.65 g) in THF (40 mL) was added 60% sodium hydride (0.24 g) under ice-cooling. The reaction mixture was stirred at room temperature for 2 hr, and the solvent was evaporated under reduced pressure. Water was added and the mixture was extracted with a mixed solvent of ethyl acetate-THF, and the extract was washed with saturated aqueous sodium hydrogen carbonate solution and saturated brine, dried over anhydrous magnesium sulfate and the residue was crystallized from ethyl acetate-isopropyl ether to give ethyl 1-methyl-8-(pyridin-4-ylmethoxy)-4,5-dihydro-1H-thieno[3,4-g]indazole-6-carboxylate (0.70 g). A mixed solution of an aqueous solution of the obtained ethyl 1-methyl-8-(pyridin-4-ylmethoxy)-4,5-dihydro-1H-thieno[3,4-g]indazole-6-carboxylate (0.56 g) and potassium hydroxide (0.20 g) in water (15 ml), and ethanol (10 ml) was refluxed under heating for 3 hr. 2N Hydrochloric acid was added and the mixture was extracted with a mixed solvent of ethyl acetate-THF. The extract was washed with saturated brine, and dried over anhydrous magnesium sulfate. The solvent was evaporated under reduced pressure. This was dissolved in THF (15 ml), oxalyl chloride (0.25 ml) was added under ice-cooling, and the mixture was stirred at room temperature for 2 hr. Thereto was added aqueous ammonia solution (2 ml) and the mixture was stirred for 30 min. The solvent was evaporated under reduced pressure. The residue was extracted with ethyl acetate, and the extract was washed with saturated aqueous sodium hydrogen carbonate solution and saturated brine, and dried over anhydrous magnesium sulfate. The solvent was evaporated under reduced pressure. The residue was purified by silica gel column chromatography (methanol/ethyl acetate) to give the title compound (0.22 g). ¹H NMR (300 MHz, DMSO-d₆) δ2.56(2H, t, J=7.2 Hz), 3.12(2H, t, J=7.4 Hz), 3.95(3H, s), 5.44(2H, s), 7.32(1H, s), 7.52(2H, d, J=5.8 Hz), 7.70(1H, brs), 7.97(1H,brs), 8.64(2H, d, J=6.0 Hz)

### Reference Example 6

### N,1-dimethyl-8-phenoxy-4,5-dihydro-1H-thieno[3,4-g]indazole-6-carboxamide

To a solution (6 ml) of 1-methyl-8-phenoxy-4,5-dihydro-1H-thieno[3,4-g]indazole-6-carboxylic acid (1.29 g) in THF were added oxalyl chloride (0.7 ml) and DMF (2 drops) under ice-cooling, and the mixture was stirred at room temperature for 30 min and concentrated under reduced pressure. The residue was suspended in THF (30 ml), 40% methylamine solution (6 ml) was added under ice-cooling, and the mixture was stirred at room temperature for 1 hr, concentrated and extracted with ethyl acetate. The extract was washed with water and saturated brine, and dried over anhydrous magnesium sulfate. The residue was purified by silica gel column chromatography (ethyl acetate/hexane) to give the title compound (1.24 g).
¹H NMR (200 MHz, DMSO-d₆) δ 2.62 (2 H, t, J=6.7 Hz), 2.70 (3 H, d, J=4.4 Hz), 3.05 (2 H, t, J=6.8 Hz), 3.94 (3 H, s), 7.23 - 7.31 (3 H, m), 7.35 (1 H, s), 7.43 - 7.51 (2 H, m), 7.93-7.96 (1 H, m).

### Reference Example 7

### 1-methyl-8-(pyridin-3-yloxy)-4,5-dihydro-1H-thieno[3,4-g]indazole-6-carboxamide

### Reference Example 8

### 1-methyl-8-[4-(trifluoromethoxy)phenoxy]-4,5-dihydro-1H-thieno[3,4-g]indazole-6-carboxamide

### Reference Example 9

### 8-(1,3-benzodioxol-5-yloxy)-1-methyl-4,5-dihydro-1H-thieno[3,4-g]indazole-6-carboxamide

### Reference Example 10

### 1-methyl-8-[4-(quinolin-2-ylmethoxy)phenoxy]-4,5-dihydro-1H-thieno[3,4-g]indazole-6-carboxamide

### Reference Example 11

### 1-methyl-8-(propylsulfanyl)-4,5-dihydro-1H-thieno[3,4-g]indazole-6-carboxamide

### Reference Example 12

### 1-methyl-8-(phenylamino)-4,5-dihydro-1H-thieno[3,4-g]indazole-6-carboxamide

### Reference Example 13

### 1-methyl-8-(propylsulfonyl)-4,5-dihydro-1H-thieno[3,4-g]indazole-6-carboxamide

### Reference Example 14

### 8-butyl-1-methyl-4,5-dihydro-1H-thieno[3,4-g]indazole-6-carboxamide

### Reference Example 15

### 1-methyl-8-(propylamino)-4,5-dihydro-1H-thieno[3,4-g]indazole-6-carboxamide

### Reference Example 16

### 8-benzyl-1-methyl-4,5-dihydro-1H-thieno[3,4-g]indazole-6-carboxamide

### Reference Example 17

### 8-(cyclopentyloxy)-1-methyl-4,5-dihydro-1H-thieno[3,4-g]indazole-6-carboxamide

### Reference Example 18

### 1-methyl-8-(quinolin-6-yloxy)-4,5-dihydro-1H-thieno[3,4-g]indazole-6-carboxamide

### Reference Example 19

### 1-methyl-8-[4-(methylsulfonyl)phenoxy]-4,5-dihydro-1H-thieno[3,4-g]indazole-6-carboxamide

### Reference Example 20

### 8-phenoxy-4,5-dihydro-1H-thieno[3,4-g]indazole-6-carboxamide

### Reference Example 21

### 8-(3-methoxyphenoxy)-1-methyl-4,5-dihydro-1H-thieno[3,4-g]indazole-6-carboxamide

### Reference Example 22

### 8-[4-(acetylamino)phenoxy]-1-methyl-4,5-dihydro-1H-thieno[3,4-g]indazole-6-carboxamide

### Reference Example 23

### 1-methyl-8-[4-(pyridin-4-ylmethoxy)phenoxy]-4,5-dihydro-1H-thieno[3,4-g]indazole-6-carboxamide

### Reference Example 24

### {4-[(6-carbamoyl-1-methyl-4,5-dihydro-1H-thieno[3,4-g]indazol-8-yl)oxy]phenyl}acetic acid

### Reference Example 25

### 8-{4-[(2S)-2-amino-3-morpholin-4-yl-3-oxopropyl]phenoxy}-1-methyl-4,5-dihydro-1H-thieno[3,4-g]indazole-6-carboxamide

### Reference Example 26

### 8-[3-(dimethylamino)phenoxy]-1-methyl-4,5-dihydro-H-thieno[3,4-g]indazole-6-carboxamide

### Reference Example 27

### 1-methyl-8-[4-(pyridin-2-ylmethoxy)phenoxy]-4,5-dihydro-1H-thieno[3,4-g]indazole-6-carboxamide

### Reference Example 28

### 8-(1,3-benzodioxol-5-yloxy)-1-methyl-N-(pyridin-4-ylmethyl)-4,5-dihydro-1H-thieno[3,4-g]indazole-6-carboxamide

### Reference Example 29

### 8-(1,3-benzodioxol-5-yloxy)-1,4,4-trimethyl-4,5-dihydro-1H-thieno[3,4-g]indazole-6-carboxamide

### Reference Example 30

### 1-methyl-8-(quinolin-3-yloxy)-4,5-dihydro-1H-thieno[3,4-g]indazole-6-carboxamide

### Reference Example 31

### 1-methyl-8-[(6-methylpyridin-3-yl)oxy]-4,5-dihydro-1H-thieno[3,4-g]indazole-6-carboxamide

### Reference Example 32

### 8-[(2-aminopyridin-3-yl)oxy]-1-methyl-4,5-dihydro-1H-thieno[3,4-g]indazole-6-carboxamide

### Reference Example 33

### 1-methyl-8-[(1-oxidepyridin-3-yl)oxy]-4,5-dihydro-1H-thieno[3,4-g]indazole-6-carboxamide

### Reference Example 34

### 8-[(2-methoxypyridin-3-yl)oxy]-1-methyl-4,5-dihydro-1H-thieno[3,4-g]indazole-6-carboxamide

### Reference Example 35

### 8-[(6-methoxypyridin-3-yl)oxy]-1-methyl-4,5-dihydro-1H-thieno[3,4-g]indazole-6-carboxamide

### Reference Example 36

### 8-(dimethylamino)-1-methyl-4,5-dihydro-1H-thieno[3,4-g]indazole-6-carboxamide

### Reference Example 37

### 1-methyl-8-(pyrimidin-2-yloxy)-4,5-dihydro-1H-thieno[3,4-g]indazole-6-carboxamide

### Reference Example 38

### 1-methyl-8-[(6-methylpyridin-2-yl)oxy]-4,5-dihydro-1H-thieno[3,4-g]indazole-6-carboxamide

### Reference Example 39

### 8-(1,3-benzodioxol-5-ylsulfonyl)-1-methyl-4,5-dihydro-1H-thieno[3,4-g]indazole-6-carboxamide

### Reference Example 40

### 1-methyl-8-({2-[2-(2-oxopyrrolidin-1-yl)ethoxy]pyridin-3-yl}oxy)-4,5-dihydro-1H-thieno[3,4-g]indazole-6-carboxamide

### Reference Example 41

### 1-methyl-8-[(2-{2-[methyl(phenyl)amino]ethoxy}pyridin-3-yl)oxy]-4,5-dihydro-1H-thieno[3,4-g]indazole-6-carboxamide

### Reference Example 42

### 8-{[6-(2-methoxyethoxy)pyridin-3-yl]oxy}-1-methyl-4,5-dihydro-1H-thieno[3,4-g]indazole-6-carboxamide

### Reference Example 43

### 6-{4-[(6-carbamoyl-1-methyl-4,5-dihydro-1H-thieno[3,4-g]indazol-8-yl)oxy]phenoxy}hexanoic acid

### a) ethyl 6-(4-hydroxyphenoxy)hexanoate

A suspension of ethyl 6-[4-(benzyloxy)phenoxy]hexanoate (8.1 g) and 10% palladium carbon (2.8 g) in THF (80 ml) and ethanol (80 ml) was stirred under a hydrogen atmosphere at room temperature for 4 hr. The insoluble material was collected by filtration, and the filtrate was concentrated under reduced pressure. The residue was purified by silica gel column chromatography (ethyl acetate/hexane) to give the title compound (7.1 g).
¹H NMR (300 MHz, CDCl₃) δ 1.26(3H, t, J=7.0 Hz), 1.40 - 1.83(6H, m), 2.33(2H, t, J=7.2 Hz), 3.89(2H, t, J=6.4 Hz), 4.13(2H, q, J=7.0 Hz), 4.48(1H, s), 6.76(4H, s).

### b) tert-butyl 8-{4-[(6-ethoxy-6-oxohexyl)oxy]phenoxy}-1-methyl-4,5-dihydro-1H-thieno[3,4-g]indazole-6-carboxylate

To a solution of ethyl 6-(4-hydroxyphenoxy)hexanoate (3.1 g) in N-methylpyrrolidone(50 ml) was added tert-butoxy potassium (1.4 g), and the mixture was stirred at room temperature for 30 min. To this mixture was added tert-butyl 4,5-dihydro-1-methyl-8-methylsulfonyl-1H-thieno[3,4-g]indazole-6-carboxylate (3.65 g) and the mixture was stirred at 80°C for 5 hr. The reaction solution was cooled, aqueous oxalic acid solution was added, and the mixture was extracted with ethyl acetate. The extract was washed with water and saturated brine, and dried over anhydrous magnesium sulfate. The solvent was evaporated under reduced pressure. The residue was purified by silica gel column chromatography (ethyl acetate/hexane) to give the title compound (3.0 g).
¹H NMR (300 MHz, CDCl₃) δ1.26(3H, t, J=7.2 Hz), 1.53(9H, s), 1.45 - 1.90(6H, m), 2.35(2H, t, J=7.4 Hz), 2.68(2H, t, J=7.0 Hz), 3.22(2H, t, J=7.0 Hz), 3.96(2H, t, J=6.4 Hz), 4.08(3H, s), 4.14(2H, q, J=7.2 Hz), 6.90(2H, d, J=9.0 Hz), 7.13(2H, d, J=9.0 Hz), 7.35(1H, s).

### c) ethyl 6-{4-[(6-carbamoyl-1-methyl-4,5-dihydro-1H-thieno[3,4-g]indazol-8-yl)oxylphenoxylhexanoate

tert-Butyl 8-{4-[(6-ethoxy-6-oxohexyl)oxy]phenoxy}-1-methyl-4,5-dihydro-1H-thieno[3,4-g]indazole-6-carboxylate (3.1 g) and 6N hydrochloric acid (30 ml) were added to a mixed solution of THF (50 ml) and ethanol (10 ml) and the mixture was stirred at 50 - 60°C for 6 hr. The reaction solution was concentrated under reduced pressure, and the residue was washed with a mixed solution of ethyl acetate and hexane to give a mixture (1.95 g) of 8-{4-[(6-ethoxy-6-oxohexyl)oxy]phenoxy}-1-methyl-4,5-dihydro-1H-thieno[3,4-g]indazole-6-carboxylic acid and 8-{4-[(5-carboxypentyl)oxy]phenoxy}-1-methyl-4,5-dihydro-1H-thieno[3,4-g]indazole-6-carboxylic acid. To a solution of this mixture and ammonia-HOBt complex (1.43 g) were dissolved in DMF (40 ml), WSC (1.81 g) was added to the resulting solution under ice-cooling, and the mixture was stirred at room temperature for 20 hr. The solvent was evaporated under reduced pressure. To the residue were added water and ethyl acetate, the insoluble material was filtered off, and the organic layer was separated from the filtrate, washed with saturated aqueous sodium hydrogen carbonate solution and saturated brine, and dried over anhydrous magnesium sulfate. The solvent was evaporated under reduced pressure. The residue was purified by silica gel column chromatography (ethyl acetate/hexane) and recrystallized from THF-ethyl acetate to give the title compound (0.43 g).
¹H NMR (300 MHz, CDCl₃) δ1.26(3H, t, J=7.2 Hz), 1.44 - 1.92(6H, m), 2.35(2H, t, J=7.2 Hz), 2.71(2H, t, J=7.0 Hz), 3.18(2H, t, J=7.0 Hz), 3.95(2H, t, J=6.2 Hz), 4.09(3H, s), 4.14(2H, q, J=7.2 Hz), 5.48(2H, s), 6.89(2H, d, J=9.2 Hz), 7.13(2H, d, J=9.2 Hz), 7.36(1H, s).

### d) 6-{4-[(6-carbamoyl-1-methyl-4,5-dihydro-1H-thieno[3,4-g]indazol-8-yl)oxy]phenoxy}hexanoic acid

Ethyl 6-{4-[(6-carbamoyl-1-methyl-4,5-dihydro-1H-thieno[3,4-g]indazol-8-yl)oxy]phenoxy}hexanoate (0.40 g) was dissolved in a mixture of THF (10 ml) and methanol (10 ml), and an aqueous solution of potassium hydroxide (0.18 g) in water (5 ml) was added. The reaction solution was stirred at room temperature for 18 hr, poured into ethyl acetate and aqueous oxalic acid solution, and the organic layer was separated and dried over anhydrous magnesium sulfate. The solvent was evaporated under reduced pressure, and the residue was washed with ethyl acetate-hexane to give the title compound (0.38 g).
¹H NMR (300 MHz, DMSO-d₆) δ 1.32 - 1.82(6H, m), 2.24(2H, t, J=7.2 Hz), 2.61(2H, t, J=7.0 Hz), 3.06(2H, t, J=7.0 Hz), 3.96(2H, t, J=6.2 Hz), 3.98(3H, s), 6.99(2H, d, J=9.2 Hz), 7.25(2H, d, J=9.2 Hz), 7.33(1H, s), 7.37(2H, brs).

### Example 1: Evaluation of cell differentiation promoting activity in myoblast

(1) Mouse-derived myoblast line C2C12 cell (Dainippon Pharmaceutical Co., Ltd.) was subcultured in a high glucose Eagle's modified Dulbecco's medium (GIBCO) added with 10% calf fetal bovine serum (GIBCO), 100 unit/mL penicillin and 100 µg/mL streptomycin (hereinafter to be referred to as a growth medium), plated at 5000 cells/cm² and cultured at 37°C, under 5% CO₂.
(2) When the cells reached subconfluence of not less than 90%, the medium was exchanged with high glucose Eagle's modified Dulbecco's medium (GIBCO) added with (i) 2% horse serum (GIBCO) and (ii) penicillin, streptomycin and a test compound (hereinafter to be referred to as a differentiation medium) and the cells were cultured for 2 days. To the control group was added DMSO solution instead of the test compound. (3) The cells were washed with PBS, 0.5% NP-40 solution was added, and the cells were lysed by freeze-thawing. The cell extract was centrifuged and the creatine kinase activity in the supernatant was measured as an index of muscle differentiation. The creatine kinase activity was measured using CPKII Test Wako (Wako Pure Chemical Industries, Ltd.) according to the manual of the kit.
The creatine kinase activity of C2C12 cell added with the compound is shown in Table 1. The numerical values in Table 1 show creatine kinase activity of the compound addition group when the creatine kinase activity without addition of the compound is 100%, and * means measurement with addition of the compound at 10 µM, ** at 0.01 µM, and others at 1 µM.

**Table 1**

| compound | creatine kinase activating action (%) | | compound | creatine kinase activating action (%) |
|---|---|---|---|---|
| Ref. Ex. 1 | 175** | | Ref. Ex. 23 | 191** |
| Ref. Ex. 2 | 175** | | Ref. Ex. 24 | 142 |
| Ref. Ex. 3 | 179 | | Ref. Ex. 25 | 180 |
| Ref. Ex. 4 | 138 | | Ref. Ex. 26 | 156 |
| Ref. Ex. 5 | 113 | | Ref. Ex. 27 | 163** |
| Ref. Ex. 6 | 164 | | Ref. Ex. 28 | 186 |
| Ref. Ex. 7 | 183** | | Ref. Ex. 29 | 183 |
| Ref. Ex. 8 | 190** | | Ref. Ex. 30 | 166** |
| Ref. Ex. 9 | 171** | | Ref. Ex. 31 | 150** |
| Ref. Ex. 10 | 193** | | Ref. Ex. 32 | 101 |
| Ref. Ex. 11 | 198 | | Ref. Ex. 33 | 156 |
| Ref. Ex. 12 | 162* | | Ref. Ex. 34 | 164 |
| Ref. Ex. 13 | 128* | | Ref. Ex. 35 | 192** |
| Ref. Ex. 14 | 151 | | Ref. Ex. 36 | 139 |
| Ref. Ex. 16 | 150** | | Ref. Ex. 37 | 129 |
| Ref. Ex. 17 | 177 | | Ref. Ex. 38 | 152 |
| Ref. Ex. 18 | 160** | | Ref. Ex. 39 | 176 |
| Ref. Ex. 19 | 183 | | Ref. Ex. 40 | 120 |
| Ref. Ex. 20 | 121 | | Ref. Ex. 41 | 130 |
| Ref. Ex. 21 | 198** | | Ref. Ex. 42 | 179** |
| Ref. Ex. 22 | 195** | | | |

As shown in Table 1, in C2C12 myoblast, the test compound enhances the activity of the creatine kinase specifically expressed in muscle cell, that is, promoted the muscle cell differentiation.

### Example 2: Evaluation of sugar metabolism promoting activity in muscle cell

(1) In the same manner as in Example 1, C2C12 cell was cultured in a growth medium. After reaching subconfluence of not less than 90%, the medium was exchanged with a differentiation medium and the cells were cultured for about 7 days while exchanging the medium every 2 or 3 days until the cells differentiated to form a myotube.
(2) After confirmation of myotube formation under a microscope, the test compound (1 µM) was added to a differentiation medium and cultured for 2 days. The cells were washed with Krebs-Ringer HEPES phosphate buffer (10 mM HEPES, pH 7.4, 131 mM NaCl, 4.7 mM KCl, 2.5 mM CaCl₂-2H2O, 1.2 mM MgSO₄-7H₂O, 1.2 mM KH₂PO₄), the medium was exchanged with Krebs-Ringer HEPES phosphate buffer added with 5 mM glucose, 0.2% fatty acid-free bovine serum albumin, and cultured at 37°C for 4 hr.
(3) The lactic acid concentration of the culture supernatant was measured using Detarminer LA kit (Kyowa Medex Co., Ltd.).
Table 2 shows the concentration of lactic acid in the culture supernatant of the C2C12 cell added with the compound. The numerical values in Table 2 show the concentration of the compound addition group with the concentration of lactic acid without addition of a drug as 100%.

**Table 2**

| compound | lactic acid production promoting action (%) | | compound | lactic acid production promoting action (%) |
|---|---|---|---|---|
| Ref. Ex. 1 | 247 | | Ref. Ex. 23 | 252 |
| Ref. Ex. 2 | 159 | | Ref. Ex. 24 | 126 |
| Ref. Ex. 3 | 170 | | Ref. Ex. 25 | 235 |
| Ref. Ex. 4 | 239 | | Ref. Ex. 26 | 175 |
| Ref. Ex. 5 | 124 | | Ref. Ex. 27 | 198 |
| Ref. Ex. 6 | 187 | | Ref. Ex. 28 | 170 |
| Ref. Ex. 7 | 254 | | Ref. Ex. 29 | 264 |
| Ref. Ex. 8 | 274 | | Ref. Ex. 30 | 260 |
| Ref. Ex. 9 | 196 | | Ref. Ex. 31 | 253 |
| Ref. Ex. 10 | 259 | | Ref. Ex. 32 | 295 |
| Ref. Ex. 11 | 239 | | Ref. Ex. 33 | 155 |
| Ref. Ex. 13 | 154 | | Ref. Ex. 34 | 249 |
| Ref. Ex. 14 | 265 | | Ref. Ex. 35 | 228 |
| Ref. Ex. 16 | 194 | | Ref. Ex. 36 | 211 |
| Ref. Ex. 17 | 141 | | Ref. Ex. 37 | 255 |
| Ref. Ex. 18 | 220 | | Ref. Ex. 38 | 157 |
| Ref. Ex. 19 | 209 | | Ref. Ex. 39 | 207 |
| Ref. Ex. 20 | 167 | | Ref. Ex. 40 | 120 |
| Ref. Ex. 21 | 184 | | Ref. Ex. 41 | 120 |
| Ref. Ex. 22 | 311 | | Ref. Ex. 42 | 319 |

As shown in Table 2, the test compound promoted lactic acid production, i.e., glycolysis, in differentiated C2C12 muscle cells. Promotion of glycolysis in muscle cells leads to an enhanced sugar consumption, which is considered useful for the exhibition of a hypoglycemic action required for therapeutic drugs for diabetes.

### Example 3: Evaluation of muscle-related gene expression promoting activity of myoblast

(1) In the same manner as in Example 1, C2C12 cell was cultured, and the compound of Reference Example 1 (10 nM) was added. A DMSO solution was added to the control group instead of the test compound.
(2) One day after the addition of the test compound or DMSO solution, total RNA was extracted using Rneasy 96 kit (Qiagen) and according to the manual of the kit, and cDNA was prepared using TaqMan reverse transcription reagent (Applied Biosystems).
(3) The expression level of MyoD (Mm00440387_m1, TaqMan Gene Expression Assays, Applied Biosystems), myogenin (Mm00446194_m1, TaqMan Gene Expression Assays, Applied Biosystems), creatine kinase (CKM) (Mm00432556_m1, TaqMan Gene Expression Assays, Applied Biosystems), EGLN3 (Mm00472200_m1, TaqMan Gene Expression Assays, Applied Biosystems) was measured by quantify PCR using ABI PRISM 7900 (Applied Biosystems). The expression level of each gene, shown in Table 3, Table 4, Table 5 and Table 6, was obtained by amendment against the expression level of 36B4 (Takeda Pharmaceutical Company Limited), and is shown in a relative value to the value of the control group cultured in a growth medium as 1.
The data of Table 3, Table 4, Table 5 and Table 6 show mean±standard deviation at n=6 (n is the number of samples). GM shows cells cultured in a growth medium, DM shows cells cultured in a differentiation medium.
The following depicts the sequences of genes used for the amendment.
36B4-F: 5'-TGC CAC ACT CCA TCA TCA ATG-3' (SEQ ID NO: 1)
36B4-R: 5'-CGC AAA TGC AGA TGG ATC AG-3' (SEQ ID NO: 2)
5'-VIC- ACC TTC CCA CTT ACT GAA AAG GTC AAG GCC-TAMRA-3' (SEQ ID NO: 3)

MyoD/36B4

**Table 3**

| | GM | DM |
|---|---|---|
| control qroup | 1.20±0.09 | 1.00±0.14 |
| compound of Ref. Ex. 1 10 µM | 1.26±0.10 | 1.59±0.20# |

| | | |
|---|---|---|
| n=6, #: p≤0.05 vs. control group (t-test) | | |

Myogenin/36B4

**[Table 4]**

| | GM | DM |
|---|---|---|
| control group | 0.02±0.01 | 1.00±0.18 |
| compound of Ref. Ex. 1 10 µM | 0.03±0.00 | 3.81±0.55# |

| | | |
|---|---|---|
| n=6, #: p≤0.05 vs. control group (t-test) | | |

CKM/36B4

**[Table 5]**

| | GM | DM |
|---|---|---|
| control group | 0.03±0.01 | 1.00±0.18 |
| compound of Ref. Ex. 1 10 µM | 0.03±0.01 | 4.84±1.08# |

| | | |
|---|---|---|
| n=6, #: p≤0.05 vs. control group (t-test) | | |

EGLN3/36B4

**[Table 6]**

| | GM | DM |
|---|---|---|
| control group | 1.22±0.11 | 1.00±0.16 |
| compound of Ref. Ex. 1 10 µM | 2.12±0.15 | 2.07±1.15# |

| | | |
|---|---|---|
| n=6, #: p≤0.05 vs. control group (t-test) | | |

It was confirmed from the gene expression that the compound of Reference Example 1 increased expression of MyoD, which is a muscle cell differentiation regulating factor, in C2C12 cells cultured in a differentiation medium, increased the expression of EGLN3 said to inhibit NF-kB to promote muscle differentiation, promoted the expression of myogenin and creatine kinase, which are expressed in a muscle cell specific manner, and promoted muscle cell differentiation.

### Example 4: Evaluation of NF-kB activation suppressive action in muscle cell

(1) C2C12 cells (C2C12/NF-kB-luc, Panomics) introduced with NF-kB reporter plasmid was plated at 20000 cells/cm², 100 µg/ml hygromycin was added and the cells were cultured in a growth medium in the same manner as in Example 1.
(2) The cells were cultured in a differentiation medium added with the compound of Reference Example 1 and Reference Example 7 for 1 hr, TNF-α was added at 10 ng/mL and the cells were cultured for 4 hr.
(3) The luciferase activity in the medium was measured using a Stedy-Glo luciferase assay system (Promega). The NF-kB activity was evaluated by measuring the luciferase activity.
Table 7 and Table 8 show the activity when luciferase activity without addition of TNF-α is 1. The data in Table 7 and Table 8 show mean±standard deviation at n=8 (n is the number of samples).

**[Table 7]**

| | | | | | | | | | | | |
|---|---|---|---|---|---|---|---|---|---|---|---|
| compound of Ref. Ex. 1 (nM) | - | 30 | - | 0 | 0 | 0 | 0 | 1 | 3 | 10 | 30 |
| TNF-α (ng/mL) | - | - | 10 | 10 | 10 | 10 | 10 | 10 | 10 | 10 | 10 |
| NF-kB activity (multiple number) | 1.0 | 1.0 | 13.3 | 12.0 | 12.6 | 12.7 | 11.8 | 10.1 | 9.2 | 8.6 | 8.5 |
| SD | 0.2 | 0.2 | 1.9 | 1.3 | 1.5 | 1.0 | 1.3 | 1.0 | 0.7 | 1.0 | 0.8 |

| | | | | | | | | | | | |
|---|---|---|---|---|---|---|---|---|---|---|---|
| n=8, *: p≤0.025 vs. control group (Williams test) | | | | | | | | | | | |

**[Table 8]**

| | | | | | | | | | | | |
|---|---|---|---|---|---|---|---|---|---|---|---|
| compound of Ref. Ex. 7 (nM) | - | 1000 | - | 0 | 1 | 3 | 10 | 30 | 100 | 300 | 1000 |
| TNF-α (ng/mL) | - | - | 10 | 10 | 10 | 10 | 10 | 10 | 10 | 10 | 10 |
| NF-kB activity (multiple number) | 1.0 | 1.0 | 11.5 | 10.9 | 11.0 | 9.7 | 8.1 | 7.8 | 7.7 | 7.1 | 7.6 |
| SD | 0.2 | 0.1 | 1.0 | 1.2 | 0.8 | 1.0 | 1.3 | 1.2 | 0.9 | 0.8 | 0.9 |

| | | | | | | | | | | | |
|---|---|---|---|---|---|---|---|---|---|---|---|
| n=8, *: p≤0.025 vs. control group (Williams test) | | | | | | | | | | | |

As shown in Table 7 and Table 8, the NF-kB activity induced by TNF-α was suppressed in a concentration-dependent manner by the addition of the compounds of Reference Example 1 and Reference Example 7.

### Example 5: Evaluation of action on muscle cell differentiation suppressed by TNF-α

(1) In the same manner as in Example 1, C2C12 cells were cultured in a growth medium to not less than 90% confluence.
(2) The medium was exchanged with a differentiation medium, the compound of Reference Example 1 together with TNF-α were added, and the cells were cultured for 3 days.
(3) The cells were washed with PBS, 0.5% NP-40 solution was added, and the cells were lysed by freeze-thawing. The cell extract was centrifuged and the creatine kinase activity in the supernatant was measured as an index of muscle differentiation. The creatine kinase activity was measured using CPKII Test Wako (Wako Pure Chemical Industries, Ltd.) according to the manual of the kit.
The data in Table 9 show mean±standard deviation at n=3 (n is the number of samples).

**[Table 9]**

| | | | | | | | | |
|---|---|---|---|---|---|---|---|---|
| compound of Ref. Ex. 1 (nM) | - | - | - | - | 0 | 0 | 1 | 3 |
| TNF-α (ng/mL) | - | 3 | 10 | 30 | 30 | 30 | 30 | 30 |
| creatine kinase activity (mIU/µg) | 0.37 | 0.21 | 0.14 | 0.11 | 0.12 | 0.15 | 0.25 | 0.39 |
| SD | 0.01 | 0.01 | 0.01 | 0.02 | 0.02 | 0.03 | 0.05 | 0.03 |

| | | | | | | | | |
|---|---|---|---|---|---|---|---|---|
| n=3, *: p≤0.025 vs. control group (Williams test). #: p≤0.025 vs. TNF-α 30 (Williams test). | | | | | | | | |

As shown in Table 9, addition of TNF-α decreased creatine kinase activity in a concentration dependent manner, and the compound of Reference Example 1 recovered the creatine kinase activity decreased by TNF-α, in a concentration dependent manner.

### Example 6: Evaluation of sugar uptake promoting activity in muscle cell

(1) In the same manner as in Example 1, C2C12 cells were cultured in a growth medium to not less than 90% confluence.
(2) The medium was exchanged with a differentiation medium, and the cells were cultured until observation of myotube formation under a microscope.
(3) The compounds of Reference Example 1 and Reference Example 7 were added, and the cells were cultured overnight.
(4) The cells were washed with krebs-ringer HEPES phosphate buffer added with 1% bovine serum albumin, cultured for 30 min in this buffer, 2-deoxyglucose (2DG) (0.1 mM, 0.8 µCi/250 µL) was added and the cells were cultured at 37°C for 75 min.
(5) The cells were washed 4 times with PBS, MicroScint-20 (125 µL/well, PerkinElmer) was added and the radioactivity was measured by a scintillation counter. DMSO (dimethyl sulfoxide) alone was added to the control group. The numerical values of 2DG uptake in Table 10 are relative values to the control group as 100%.
The data in Table 10 show mean±standard deviation at n=6 (n is the number of samples).

**[Table 10]**

| | control group (DMSO) | compound of Ref. Ex. 1 (nM) | | | | compound of Ref. Ex. 7 (nM) | | | |
|---|---|---|---|---|---|---|---|---|---|
| | 0 | 0.1 | 1 | 10 | 100 | 0.1 | 1 | 10 | 100 |
| 2DG uptake (%) | 100 | 89 | 150 | 166 | 187 | 69 | 100 | 137 | 181 |
| SD | 25 | 20 | 27 | 31 | 26 | 16 | 16 | 24 | 34 |

| | | | | | | | | | |
|---|---|---|---|---|---|---|---|---|---|
| n=6, *: p≤0.025 vs. control group (Williams test) | | | | | | | | | |

As shown in Table 10, the compounds of Reference Example 1 and Reference Example 7 promoted sugar uptake of C2C12 cell, differentiated into muscle cell, in a dose-dependent manner. Promotion of sugar uptake in muscle cells leads to an enhanced sugar consumption, which is considered useful for the exhibition of a blood glucose-lowering action required for therapeutic drugs for diabetes.

### Example 7: Evaluation of antiobesity action and dyslipidemia improving effect in high-fat diet-loaded rat

Male FisherF344 rats (CLEA Japan, Inc.) fed with a high-fat diet (45 kcal% fat, D12451, research diet) for 38 weeks from 5 weeks of age were used. They were bred with a powder high-fat diet (D12451M) for 4 weeks for acclimation, measured for body weight, body composition, amount of ingestion and plasma component, and divided into 3 groups (6 per group) without difference between them. The plasma components (total cholesterol, triglyceride, glucose concentration) were measured by HITACHI automatic analyzer 7070. As the body composition, total fat mass, body fat percentage, lean body mass of lower leg, and visceral fat mass were measured by X-ray CT apparatus (LCT-100A, LaTheta). The compound of Reference Example 1 was suspended in aqueous methylcellulose solution and orally administered repeatedly to 2 groups of the rats divided into 3 groups at 0.03 mg/kg or 0.1 mg/kg once a day for 14 days. A 0.5% aqueous methylcellulose solution was orally administered similarly to the remaining one group and used as a control group. On the administration completion day, the blood was drawn again from the tail vein, and plasma levels of total cholesterol, triglyceride, glucose, insulin and leptin, and body weight and body composition (total fat mass, body fat percentage, lean body mass of lower leg, visceral fat mass) were measured. The amount of ingestion was calculated from the amount of the feed given and the amount left, and the total of the test period was calculated. The results are shown in Table 11 and Table 12. The numerical values in Table 11 and Table 12 show mean±standard deviation at n=5-6 (n is the number of rats).

**[Table 11]**

| | control group | compound of Ref. Ex. 1 | compound of Ref. Ex. 1 |
|---|---|---|---|
| | | (0.03 mg/kg) | (0.1 mg/kg) |
| body weight (q) | 462.6±33.2 | 460.8±15.3 | 446.5±38.1 |
| amount of change (g) | -0.1±4.3 | -3.8±7.5 | -19.5±11.0* |
| total fat mass (g) | 156.8±21.8 | 138.0±16.6 | 120.5±18.2* |
| amount of change (q) | -1.5±3.4 | -17.5±8.8* | -38.1±6.7* |
| visceral fat mass (q) | 61.3±6.7 | 53.8±6.1 | 47.2±9.0* |
| amount of change (g) | 0.6±1.0 | -4.3±3.4* | -11.0±2.9* |
| lean body mass (g) of lower leg | 75.5±3.7 | 82.9±5.2* | 84.5±6.9* |
| amount of change (g) | 2.6±3.2 | 8.4±1.7* | 9.8±4.2* |
| body fat percentage (%) | 33.8±3.0 | 29.9±2.7* | 26.9±2.6* |
| change rate (%) | -0.3±0.5 | -3.5±1.5* | -7.1±1.1* |
| amount of ingestion (g/2 weeks) | 187.0±14.5 | 165.8±13.9 | 127.9±21.2 |

| | | | |
|---|---|---|---|
| *: p≤0.025 vs. control group (Williams test). | | | |

**[Table 12]**

| | control group | compound of Ref. Ex. 1 (0.03 mg/kg) | compound of Ref. Ex. 1 (0.1 mg/kg) |
|---|---|---|---|
| triglyceride (mg/dL) | 423.9±170.8 | 242.4±92.5* | 100.1±43.1* |
| total cholesterol (mg/dL) | 158.7±30.8 | 102.3±12.6* | 77.3±9.6* |
| glucose (mg/dL) | 141.8±17.9 | 142.3±5.6 | 146.3±5.9 |
| insulin (ng/mL) | 6.7±2.1 | 5.2±1.0 | 2.8±1.1* |
| leptin (ng/dL) | 2.4±0.4 | 1.2±0.4* | 0.6±0.4* |

| | | | |
|---|---|---|---|
| *: p≤0.025 vs. control (Williams test). | | | |

As shown in Table 11, the compound of Reference Example 1 showed a clear body weight decreasing action, accompanied by decrease in the amount of ingestion, body fat percentage, total fat mass and visceral fat mass, by the administration at 0.1 mg/kg. At that time, the lean body mass of the lower leg increased, which is considered to be the muscle mass. It has been clarified therefrom that obesity model rat fed with a high-fat diet shows an antiobesity effect. The plasma markers then (Table 12) showed decreased total cholesterol and triglyceride concentration, as well as improved lipid metabolism, though no change was found for the glucose concentration. It was confirmed that a compound having a muscle cell differentiation promoting or/and adipocyte differentiation suppressive action showed a superior antiobesity action and dyslipidemia improving effect, since insulin and leptin decreased.

### Example 8: Evaluation of anti-diabetes action and dyslipidemia improving effect in KKA^{y} mouse

Female KKA^{y} mice (12-week-old, CLEA Japan, Inc.) were bred while allowing free ingestion of both feed and water. The mice were bred for acclimation with powder CE-2 (CLEA Japan, Inc.) from 2 weeks before administration of the compound. The body weight and plasma markers were measured and, at 14 weeks of age, they were divided such that the measured values were uniform with those of the control group. The compound of Reference Example 1, 2, 7, 8, 9 or 22 was administered for 2 weeks in a mixed feed (0.01-0.0001%). On the last day, plasma levels of glucose, insulin, total cholesterol and neutral lipid were measured. The numerical values in Table 13 are relative values to each measured value of the control group as 100, and show mean±standard deviation at n=5 or 6, NT shows not tested, and n shows the number of samples.

**[Table 13]**

| compound | mixed feed dose (%) | glucose (%) | neutral lipid (%) | total cholesterol (%) | insulin (%) |
|---|---|---|---|---|---|
| Ref. Ex. 1 | 0.0001 | 53.2±19.1 | 38.9±11.0 | 61.6±10.8 | 27.3±19.5 |
| Ref. Ex. 7 | 0.01 | 40.3±4.5 | 33.9±8.6 | 64.8±12.0 | 2.9±1.7 |
| Ref. Ex. 2 | 0.0003 | 49.0±7.1 | 40.4±6.6 | 62.4±7.1 | NT |
| Ref. Ex. 8 | 0.0003 | 78.2±22.2 | 72.2±22.1 | 85.7±11.5 | NT |
| Ref. Ex. 9 | 0.001 | 42.9±3.7 | 51.0±11.9 | 74.5±14.4 | 13.7±21.1 |
| Ref. Ex. 22 | 0.0003 | 60.9±27.1 | 42.3±7.2 | 71.0±7.3 | NT |

As shown in Table 13, the compounds lowered the plasma glucose concentration, plasma neutral lipid concentration, plasma total cholesterol concentration and plasma insulin concentration of KKA^{y} mice.
From the above results, it was confirmed that a compound having a muscle cell differentiation promoting or/and adipocyte differentiation suppressive action has a superior anti-diabetes action and dyslipidemia improving effect.

### Example 9: Evaluation of improvement of insulin resistance in KKA^{y} mouse

Female KKA^{y} mice (12-week-old, CLEA Japan, Inc.) were bred while allowing free ingestion of both feed and water. The mice were bred for acclimation with powder CE-2 (CLEA Japan, Inc.) from 2 weeks before administration of the compound. The body weight and plasma markers were measured and, at 14 weeks of age, they were divided into 4 groups (6 per group) such that the measured values were uniform. The compound of Reference Example 1 was administered for 4 weeks in a mixed feed (0.01-0.09 mg/kg/day). After fasting overnight, insulin was intraperitoneally administered at 0.5 U/kg and, after 30, 60 and 120 min, blood samples were collected. The plasma glucose concentration was measured, and the area under the plasma glucose concentration time curve (AUC) was calculated. n shows the number of samples.

**[Table 14]**

| | glucose AUC_{0-120 min} (mg h/dL) | SD |
|---|---|---|
| control group | 265.9 | 31.9 |
| compound of Ref. Ex. 1 0.01 mg/kg/day | 241.0 | 26.2 |
| compound of Ref. Ex. 1 0.03 mg/kg/day | 184.7* | 36.0 |
| compound of Ref. Ex. 1 0.09 mg/kg/day | 182.5* | 54.7 |

| | | |
|---|---|---|
| n=5-6, *: p≤0.025 vs. control (Williams test) | | |

As shown in Table 14, the area under the plasma glucose concentration time curve after insulin administration decreased in the administration group, showing that insulin resistance was improved.

### Example 10: Evaluation of adipocyte differentiation suppressive action

C3H10T1/2 cells (Dainippon Pharmaceutical Co., Ltd.) were cultured in a growth medium until confluence. 1 µM dexamethasone, 0.5 mM 3-isobutyl-1-methylxanthine and 5 µg/mL insulin were added to the medium, and the cells were cultured for 3 days. The medium was exchanged with a growth medium added with 4 µg/mL insulin and the cells were cultured for 3 more days and cultured in a growth medium for 3 days. When the compound was added, it was added for 9 days after reaching confluence and the cells were cultured. The cells were fixed with 3.7% formalin, lipid droplet was stained with 100 nM BODIPY 493/503 (Molecular Probes), nucleus was stained with 2 µM Hoechst33258 (Acros Organics), and analyzed by IN Cell analyzer (GE Healthcare). The data of Table 15 show mean±standard deviation at n=4 (n is the number of samples). GM shows cells cultured in a growth medium, DM shows cells cultured in a differentiation medium.

**[Table 15]**

| medium | GM | DM | DM | DM | DM |
|---|---|---|---|---|---|
| compound | control group | control group | compound of Ref. Ex. 1 (nM) | | |
| concentration (nM) | - | - | 0.1 | 1 | 10 |
| lipid droplet area (lipid droplet stained area/cell number) | 448 ±334 | 11771 ±3680 | 9625 ±2657 | 5703 ±1417* | 2010 ±703* |
| cell number (number of nucleus/visual field) | 371±38 | 305±28 | 303±28 | 295±15 | 318±9 |

| | | | | | |
|---|---|---|---|---|---|
| n=4, *: p≤0.025 vs. control group (Williams test) | | | | | |

As shown in Table 15, in adipocyte differentiation, the compound of Reference Example 1 decreased the intracellular lipid accumulation in a concentration dependent manner without affecting the cell number and has an adipocyte differentiation suppressive action.

### Example 11: Influence on adipocyte-related gene expression level

In the same manner as in Example 10, the cells were cultured, and total RNA was extracted using Rneasy96 kit (Qiagen) from the cell immediately before and 6 days after addition of 10 µm of the compound of Reference Example 1. cDNA was prepared from the total RNA using TaqMan reverse transcription reagent (Applied Biosystems), quantitative PCR was performed by ABI PRISM7000 or 7000 using TaqMan Universal PCR master mix (Applied Biosystems) and commercially available primers, and the expression level of each gene was determined. The commercially available primers used were as follows.
C/EBPα (Mm00514283_s1, TaqMan Gene Expression Assays, Applied Biosystems)
C/EBPβ (Mm00843434_s1, TaqMan Gene Expression Assays, Applied Biosystems)
KLF15 (Mm00517792_m1, TaqMan Gene Expression Assays, Applied Biosystems)
PPARγ (Mm00440945_m1, TaqMan Gene Expression Assays, Applied Biosystems)
The expression level was amended by the amount of 36B4 (Takeda Pharmaceutical Company Limited) in the same manner as in Example 3, and the relative values to the value immediately before addition of each gene compound as 1 are shown in Table 16. The data in Table 16 show mean±standard deviation at n=3-4 (n is the number of samples).

**[Table 16]**

| gene | control group | compound of Ref. Ex. 1 |
|---|---|---|
| C/EBP**α** | 3.2±0.7 | 1.6±0.5 |
| C/EBP**β** | 3.6±0.4 | 1.3±0.5 |
| KLF15 | 3.0±0.4 | 1.4±0.4 |
| PPAR**γ** | 8.0±1.4 | 4.5±0.9 |

As shown in Table 16, in C3H10T1/2 cell, the compound of Reference Example 1 decreased the expression levels of C/EBPα, C/EBPβ, KLF15 and PPARγ, which are known to be important for adipocyte differentiation, thus exhibiting suppression of adipocyte differentiation.

### Example 12: Influence on ATP content and succinic acid dehydrogenase activity in muscle cell

(1) In the same manner as in Example 1, C2C12 cells were cultured in a growth medium to not less than 90% confluence.
(2) The medium was exchanged with a differentiation medium, and the cells were cultured until observation of myotube formation under a microscope.
(3) The compounds of Reference Example 1 and Reference Example 7 were added, and the cells were cultured for two days.
(4) ATP content was measured using Cell Titer-GloTM Luminescence cell viable assay (Promega) and succinic acid dehydrogenase activity of mitochondria was measured using Cell Counting Kit-8 (Dojin). The data of Table 17 show mean±standard deviation at n=3-4 (n is the number of samples).

**[Table 17]**

| | control group | compound of Ref. Ex. 1 (nM) | | | compound of Ref. Ex. 7 (nM) | | |
|---|---|---|---|---|---|---|---|
| | | 0.01 | 0.1 | 1 | 1 | 10 | 100 |
| ATP content | 100±4 | 108±1* | 107±0* | 112±1* | 106±2* | 111±3* | 103±4* |
| succinic acid dehydrogenase activity | 100±2 | 98±10 | 113±5* | 124±3* | 111±3* | 120±4* | 131±7* |

| | | | | | | | |
|---|---|---|---|---|---|---|---|
| *: p≤0.025 vs. control group (Williams test). | | | | | | | |

As shown in Table 17, the compounds of Reference Example 1 and Reference Example 7 did not decrease the ATP content of muscle cells but increased succinic acid dehydrogenase activity, which is a mitochondrial marker.

### Example 13: Evaluation of antiobesity action, dyslipidemia improving effect, and diabetes improving effect in mouse fed with high-fat diet

Male C57BL/6J mice (CLEA Japan, Inc.) were bred from 5-week-old on a high-fat diet (D12451, research diet) while allowing free ingestion of both feed and water to prepare obesity mice. The mice were bred for acclimation for 2 weeks from 21-week-old. The body weight and plasma markers were measured when 23-week-old, the mice were divided into 6 groups (6 per group) such that the values thereof were uniform, and Reference Example 1 (0.01-0.1 mg/kg) or Reference Example 7 (1-3 mg/kg) was orally administered for 5 weeks. After 14 and 28 days, plasma levels of total cholesterol, neutral lipid, glucose, insulin, leptin, adiponectin and glycated hemoglobin were measured. On the last day, the mice were autopsied, and weights of mesenteric fat, epididymal fat, liver and gastrocnemius muscle were measured. The results are shown in Table 18. The data of Table 18 show mean±standard deviation at n=5-6 (n is the number of samples).

**[Table 18]**

| | | control group | compound of Ref. Ex. 7 | | compound of Ref. Ex. 1 | | |
|---|---|---|---|---|---|---|---|
| | | | 1 mg/kg | 3 mg/kg | 0.01 mg/kg | 0.03 mg/kg | 0.1 mg/kg |
| body weight (g) | Day 0 | 40.5±2.1 | 41.2±1.8 | 41.1±1.7 | 40.9±2.5 | 40.9±2.2 | 38.8±3.5 |
| | Day 37 | 46.3±2.4 | 45.6±3.0 | 44.0±2.4 | 44.9±2.6 | 41.0±2.3* | 35.6±3.9* |
| | change | 5.4±1.1 | 4.0±1.5 | 2.3±2.3* | 3.0±1.2* | -0.2±0.9* | -4.1±0.7* |
| average amount of ingestion (g/day) | | 3.8±0.3 | 3.6±0.2 | 3.7±0.2 | 3.7±0.2 | 3.2±0.2* | 2.7±0.3* |
| plasma total cholesterol (mg/dL) | Day 0 | 244.0±39.7 | 227.2±21.2 | 231.9±29.9 | 222.1±45.4 | 238.2±19.0 | 218.2±19.0 |
| | Day 14 | 239.0±21.2 | 213.8±13.0* | 192.8±12.5* | 182.6±19.5* | 165.6±11.3* | 129.1±15.4* |
| | Day 28 | 235.0±22.9 | 216.8±20.7 | 194.1±18.4* | 187.2±15.9* | 170.3±13.5* | 135.1±15.4* |
| plasma neutral fats (mg/dL) | Day 0 | 66.4±35.6 | 69.8±35.0 | 71.6±28.8 | 73.4±15.1 | 72.3±34.0 | 63.2±9.1 |
| | Day 14 | 85.9±18.7 | 68.8±16.0 | 47.0±9.2* | 59.4±18.5 | 50.9±14.7* | 25.2±7.6* |
| | Day 28 | 62.6±23.0 | 62.1±26.1 | 53.6±35.6 | 45.5±14.1 | 54.4±19.8 | 34.7±12.5* |
| plasma glucose (mg/dL) | Day 0 | 181.0±22.8 | 168.4±29.1 | 176.9±30.1 | 178.1±26.5 | 178.5±27.8 | 183.9±35.2 |
| | Day 14 | 157.9±8.0 | 157.5±19.4 | 172.8±32.3 | 156.0±23.4 | 154.9±12.1 | 159.6±26.7 |
| | Day 28 | 165.6±28.3 | 149.7±16.9 | 155.9±12.7 | 162.9±24.4 | 167.0±20.4 | 138.6±17.3 |
| plasma insulin (ng/mL) | Day 0 | 3.7±1.7 | 5.3±3.5 | 5.1±2.3 | 4.2±2.0 | 6.0±3.7 | 4.3±1.8 |
| | Day 14 | 11.3±4.4 | 7.8±3.3 | 6.2±3.0* | 6.2±5.8 | 3.5±2.1* | 1.4±0.6* |
| | Day 28 | 17.1±9.3 | 12.3±4.9 | 5.8±1.4* | 5.4±3.9* | 2.7±0.8* | 1.4±0.6* |
| plasma leptin (mg/dL) | Day 0 | 57.3±15.2 | 61.4±18.1 | 62.3±10.9 | 54.2±21.0 | 70.3±19.6 | 55.7±15.4 |
| | Day 14 | 92.8±9.5 | 86.9±12.0 | 73.5±12.6* | 71.2±22.0 | 54.7±19.0* | 27.4±19.1* |
| | Day 28 | 99.9±11.9 | 96.1±10.2 | 77.3±16.3* | 81.8±23.9 | 62.4±18.1* | 29.2±20.0* |
| plasma adiponectin (µg/dL)) | Day 0 | 19.1±2.5 | 18.3±2.8 | 17.0±2.9 | 15.9±3.4 | 18.0±4.0 | 16.8±5.7 |
| | Day 14 | 14.5±4.6 | 14.9±3.4 | 15.2±3.0 | 15.5±2.5 | 19.5±3.1* | 22.8±3.8* |
| | Day 28 | 12.8±1.5 | 12.2±2.3 | 12.8±0.8 | 14.7±2.9 | 16.4±3.8 | 19.2±2.9* |
| glycated hemoglobin (%) | Day 0 | 4.5±0.2 | 4.4±0.1 | 4.6±0.1 | 4.5±0.2 | 4.5±0.1 | 4.5±0.2 |
| | Day 14 | 4.4±0.1 | 4.2±0.2 | 4.0±0.3 | 4.0±0.3* | 4.1±0.1* | 4.0±0.2* |
| | Day 28 | 4.1±0.2 | 4.0±0.1 | 4.0±0.2 | 3.8±0.4 | 4.1±0.1 | 3.8±0.2* |
| mesenteric fat mass (g) | Day 38 | 1.11±0.19 | 0.95±0.14 | 0.77±0.24* | 0.87±0.17* | 0.57±0.11* | 0.30±0.08* |
| epididymal fat mass (g) | Day 38 | 0.82±0.19 | 0.78±0.11 | 0.77±0.15 | 0.91±0.26 | 0.72±0.05 | 0.47±0.10* |
| gastrocnemius muscle weight (g/100 g body weight) | Day 38 | 0.357±0.021 | 0.360±0.014 | 0.375±0.020 | 0.373±0.030 | 0.407±0.017* | 0.468±0.033* |
| hepatocyte weight (g) | Day 38 | 2.06±0.49 | 1.85±0.30 | 1.74±0.30 | 1.81±0.23 | 1.56±0.11* | 1.37±0.24* |

| | | | | | | | |
|---|---|---|---|---|---|---|---|
| *: p≤0.025 vs. control group (Williams test). | | | | | | | |

As shown in Table 18, in obese mouse model fed with a high-fat diet, the compounds of Reference Examples 1 and 7 showed a clear body weight decreasing action, accompanied by decrease in the amount of ingestion, visceral fat, epididymal fat and hepatic tissue weight. At that time, the skeletal muscle mass (gastrocnemius muscle weight per 100 g body weight) increased. It has been clarified therefrom that obesity model rat fed with a high-fat diet shows an antiobesity effect. The plasma markers then showed improvement of glucose and lipid metabolism associated with decreases in glycated hemoglobin value, total cholesterol and triglyceride concentration, though no change was found for the glucose concentration. In addition, since plasma insulin concentration decreased, the possibility of improvement in the insulin resistance was considered. It was confirmed that a compound having a muscle cell differentiation promoting or/and adipocyte differentiation suppressive action showed a superior antiobesity action and dyslipidemia, diabetes improving effect also in mouse.

### Example 14: data of normal mouse

Normal male C57BL/6J mice (CLEA Japan, Inc.) were bred allowing free ingestion of both feed and water. The mice were bred for acclimation for 1 week from 16-week-old. The body weight was measured, the mice were divided into 2 groups (5 per group) to be uniform, and the compound of Reference Example 1 (0.03 mg/kg) was orally administered to one group for 2 weeks. On the last day, the body weight, and plasma levels of total cholesterol, glucose, lactic acid, GOT, GPT and neutral lipid were measured. The results are shown in Table 19. The data of Table 19 show mean±standard deviation at n=5 (n is the number of samples).

**[Table 19]**

| | control group | compound of Ref. Ex. 1 |
|---|---|---|
| | | 0.03 mg/kg |
| body weight (g) on last day | 28.27±1.25 | 28.03±0.84 |
| total amount of ingestion (g/2 weeks) | 57.1±3.9 | 55.6±2.5 |
| plasma total cholesterol (mg/dL) | 85.4±7.2 | 81.1±10.2 |
| plasma neutral fats (mg/dL) | 79.9±17.7 | 78.0±31.0 |
| plasma glucose (mg/dL) | 147.5±14.5 | 155.5±11.9 |
| plasma lactic acid (mg/dL) | 40.4±5.8 | 49.3±16.5 |
| plasma GOT (U/L) | 50.5±7.2 | 63.4±31.1 |
| plasma GPT (U/L) | 30.7±2.4 | 29.4±3.9 |

As shown in Table 19, when the compound of Reference Example 1 was administered to normal C57BL/6J mice, decrease in body weight, amount of ingestion, plasma total cholesterol concentration, plasma neutral fats concentration and plasma glucose concentration was not found as results in the diabetic or obese mice shown in Example 8 or 13, nor affected plasma levels of lactic acid, GOT and GPT. That is, since the compound of Reference Example 1 exhibits an action only on pathology such as diabetes, obesity and dyslipidemia, and does not affect normal body weight and plasma markers, it is considered to be promising as a therapeutic drug.

### Example 15: Evaluation of phosphorylated Acetyl-CoA carboxylase (ACC) amount in muscle cell

(1) In the same manner as in Example 1, C2C12 cells were cultured in a growth medium. After reaching subconfluence of not less than 90%, the medium was exchanged with a differentiation medium and the cells were cultured for about 4 days while exchanging the medium every 2 or 3 days until the cells differentiated to form a myotube.
(2) After confirmation of myotube formation under a microscope, the test compound (0.1-10 nM) was added to a differentiation medium and cultured for 3 days. Thereafter, the cells were fixed with 4% formaldehyde phosphate buffer for 10 min, and washed 4 times with PBS containing 0.1% TritonX-100.
(3) Odyssey Blocking Buffer (LI-COR Biosciences) was added, and the mixture was incubated at room temperature for 1 hr. Thereafter, primary antibody (ACCα (T-18) (Santacruz) and Phospho-ACC (Ser79) (Upstate)) were added, and the mixture was incubated at 4°C overnight.
(4) The cells were washed 4 times with PBS containing 0.1% Tween20, secondary antibody (Donkey anti-goat IRDye 800CW (LI-COR Biosciences) and Donkey anti-mouse IRDye 680 (LI-COR Biosciences)) were added and the mixture was incubated under shading at room temperature for 1 hr.
(5) The cells were washed 4 times with PBS containing 0.1% Tween20, PBS was added, and the phospho-ACC amount and total ACC amount were measured using Odyssey2.1 (LI-COR Biosciences).
Table 20 shows the phospho-ACC amount of C2C12 cell when the compound was added. The numerical values in Table 20 were obtained by amending the phospho-ACC amount with total ACC amount and are shown based on the absence of addition of the compound as 100%. n is the number of samples.

**[Table 20]**

| | | | | | | |
|---|---|---|---|---|---|---|
| compound of Ref. Ex. 1 (nM) | - | 0.1 | 0.3 | 1 | 3 | 10 |
| Phospho-ACC amount (%) | 100 | 112 | 125 | 136 | 167 | 163 |
| SD | 12 | 10 | 6 | 12 | 10 | 12 |

| | | | | | | |
|---|---|---|---|---|---|---|
| n=6 | | | | | | |

As shown in Table 20, the compound of Reference Example 1 increased phospho-ACC amount of differentiated C2C12 muscle cell. When the Phospho-ACC amount increases, the ACC activity is attenuated, and suppression of lipid synthesis and promotion of lipid combustion in the skeletal muscle are expected. Therefrom it is considered that the compound of Reference Example 1 has a fat reduction action requested for antiobesity drugs.

### Industrial Applicability

Compound (II) is useful as a drug for the prophylaxis or treatment of diabetes, obesity or dyslipidemia caused by abnormality in muscle cell differentiation and/or adipocyte differentiation. In addition, compound (II) is useful as a promoter of muscle cell differentiation and/or inhibitor of adipocyte differentiation not only for diabetes, obesity or dyslipidemia, but other diseases associated with decreased muscle mass and/or increased adipose tissue (e.g., cachexia), as well as a drug for the prophylaxis or treatment of organ disorders (e.g., steatohepatitis) associated with decreased muscle mass and/or increased adipose tissue.

This application is based on a patent application No. 2010-208852 filed in Japan, the contents of which are incorporated in full herein.

## Claims

1. An agent for the prophylaxis or treatment of diabetes, obesity or dyslipidemia, comprising a compound represented by the formula (I) 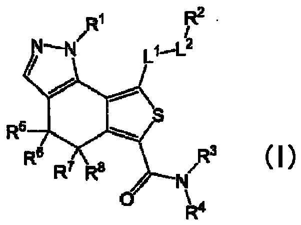 wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a Cm6 alkylene group, or a salt thereof.

2. An agent for regulating muscle cell and/or adipocyte differentiation, comprising a compound represented by the formula (I) 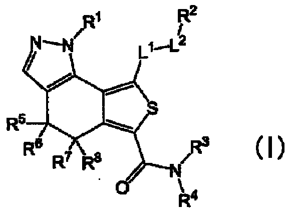 wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof.

3. The agent of claim 1 or 2, wherein R² is
(1) a C₁₋₆ alkyl group optionally substituted by 1 to 3 substituents selected from
(a) a C₁₋₆ alkoxy group, and
(b) a 5- or 6-membered aromatic heterocyclic group,
(2) a C₃₋₈ cycloalkyl group,
(3) a 5- to 10-membered aromatic heterocyclic group optionally substituted by 1 to 3 substituents selected from
(a) a C₁₋₆ alkyl group,
(b) a C₁₋₆ alkoxy group optionally substituted by 1 to 3 substituents selected from
(i) a C₁₋₆ alkoxy group,
(ii) an amino group optionally mono- or di-substituted by substituent(s) selected from a C₁₋₆ alkyl group and a phenyl group, and
(iii) a 5- or 6-membered nonaromatic heterocyclic group optionally substituted by an oxo group,
(c) an amino group, and
(d) an oxide group (-O⁻), or
(4) a phenyl group optionally substituted by 1 to 3 substituents selected from
(a) an amino group optionally mono- or di-substituted by substituent(s) selected from
(i) a C₁₋₆ alkyl group, and
(ii) a C₁₋₆ alkyl-carbonyl group optionally substituted by 1 to 3 halogen atoms,
(b) a C₁₋₆ alkylsulfonyl group,
(c) a C₁₋₆ alkoxy group optionally substituted by 1 to 3 substituents selected from
(i) a halogen atom,
(ii) a 5- to 10-membered aromatic heterocyclic group, and
(iii) a carboxy group,
(d) a halogen atom,
(e) a C₁₋₆ alkyl group optionally substituted by 1 to 3 substituents selected from
(i) a carboxy group,
(ii) an oxo group,
(iii) a 5- or 6-membered nonaromatic heterocyclic group, and
(iv) an amino group optionally mono- or di-substituted by C₁₋₆ alkyl group(s), and
(f) a C₁₋₃ alkylenedioxy group.

4. The agent of claim 1 or 2, wherein R³ is
(1) a hydrogen atom,
(2) a C₁₋₆ alkyl group optionally substituted by 1 to 3 5- or 6-membered aromatic heterocyclic groups, or
(3) a 5- or 6-membered aromatic heterocyclic group.

5. The agent of claim 1 or 2, wherein R¹ is a hydrogen atom or methyl;
R² is
(1) a C₁₋₆ alkyl group optionally substituted by 1 to 3 substituents selected from
(a) a C₁₋₆ alkoxy group, and
(b) a 5- or 6-membered aromatic heterocyclic group,
(2) a C₃₋₈ cycloalkyl group,
(3) a 5- to 10-membered aromatic heterocyclic group optionally substituted by 1 to 3 substituents selected from
(a) a C₁₋₆ alkyl group,
(b) a C₁₋₆ alkoxy group optionally substituted by 1 to 3 substituents selected from
(i) a C₁₋₆ alkoxy group,
(ii) an amino group optionally mono- or di-substituted by substituent(s) selected from a C₁₋₆ alkyl group and a phenyl group, and
(iii) a 5- or 6-membered nonaromatic heterocyclic group optionally substituted by an oxo group,
(c) an amino group, and
(d) an oxide group (-O⁻), or
(4) a phenyl group optionally substituted by 1 to 3 substituents selected from
(a) an amino group optionally mono- or di-substituted by substituent(s) selected from
(i) a C₁₋₆ alkyl group, and
(ii) a C₁₋₆ alkyl-carbonyl group optionally substituted by 1 to 3 halogen atoms,
(b) a C₁₋₆ alkylsulfonyl group,
(c) a C₁₋₆ alkoxy group optionally substituted by 1 to 3 substituents selected from
(i) a halogen atom,
(ii) a 5- to 10-membered aromatic heterocyclic group, and
(iii) a carboxy group,
(d) a halogen atom,
(e) a C₁₋₆ alkyl group optionally substituted by 1 to 3 substituents selected from
(i) a carboxy group,
(ii) an oxo group,
(iii) a 5- or 6-membered nonaromatic heterocyclic group, and
(iv) an amino group optionally mono- or di-substituted by C₁₋₆ alkyl group(s), and
(f) a C₁₋₃ alkylenedioxy group;
R³ is
(1) a hydrogen atom,
(2) a C₁₋₆ alkyl group optionally substituted by 1 to 3 5- or 6-membered aromatic heterocyclic groups, or
(3) a 5- or 6-membered aromatic heterocyclic group;
R⁴ is a hydrogen atom;
R⁵ is a hydrogen atom or methyl;
R⁶ is a hydrogen atom or methyl;
R⁷ is a hydrogen atom;
R⁸ is a hydrogen atom;
L¹ is a bond, O, S, SO₂ or NR⁹;
R⁹ is a hydrogen atom or a C₁₋₆ alkyl group; and
L² is a bond or methylene.

6. A method for the prophylaxis or treatment of diabetes, obesity or dyslipidemia in a mammal, comprising administering an effective amount of a compound represented by the formula (I) 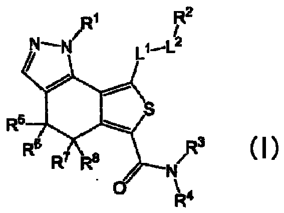 wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof to said mammal.

7. A method of regulating muscle cell and/or adipocyte differentiation in a mammal, comprising administering an effective amount of a compound represented by the formula (I) 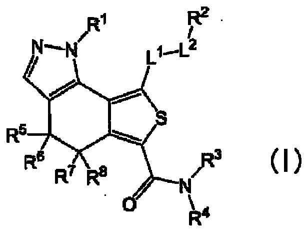 wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof to said mammal.

8. Use of a compound represented by the formula (I) 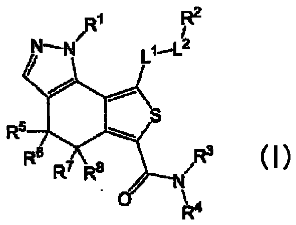 wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof for producing an agent for the prophylaxis or treatment of diabetes, obesity or dyslipidemia.

9. Use of a compound represented by the formula (I) 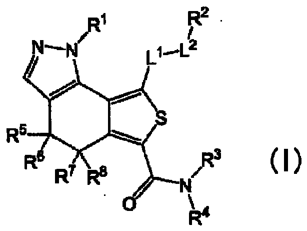 wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof for producing an agent for regulating muscle cell and/or adipocyte differentiation.

10. A compound represented by the formula (I): 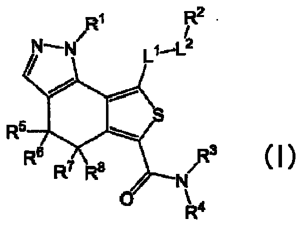 wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof for the prophylaxis or treatment of diabetes, obesity or dyslipidemia.

11. A compound represented by the formula (I): 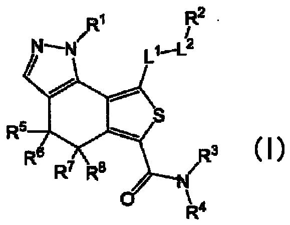 wherein
R¹ is a hydrogen atom or a C₁₋₆ alkyl group,
R² is a hydrogen atom, an optionally substituted C₁₋₆ alkyl group, an optionally substituted C₃₋₈ cycloalkyl group, an optionally substituted aromatic heterocyclic group or an optionally substituted phenyl group,
R³ is a hydrogen atom, an optionally substituted C₁₋₆ alkyl group or an optionally substituted aromatic heterocyclic group, R⁴, R⁵, R⁶, R⁷ and R⁸ are each a hydrogen atom or an optionally substituted C₁₋₆ alkyl group,
L¹ is a bond, O, S, SO, SO₂ or NR⁹,
R⁹ is a hydrogen atom or an optionally substituted C₁₋₆ alkyl group, and
L² is a bond or a C₁₋₆ alkylene group, or a salt thereof for regulating muscle cell and/or adipocyte differentiation.


SEQUENCE LISTING

<110> Takeda Pharmaceutical Company Limited

<120> THERAPEUTIC AGENT FOR DIABETES

<130> 091739

<150> JP 2010-208852
<151> 2010-09-17

<160> 3

<170> PatentIn version 3.4

<210> 1
<211> 21
<212> DNA
<213> Artificial

<220>
<223> primer

<400> 1
tgccacactc catcatcaat g 21

<210> 2
<211> 20
<212> DNA
<213> Artificial

<220>
<223> primer

<400> 2
cgcaaatgca gatggatcag 20

<210> 3
<211> 30
<212> DNA
<213> Artificial

<220>
<223> primer

<400> 3
accttcccac ttactgaaaa ggtcaaggcc 30

<110>  Takeda Pharmaceutical Company Limited

<120>  THERAPEUTIC AGENT FOR DIABETES

<130>  54 262 K

<150>  JP 2010-208852
<151>  2010-09-17

<160>  3

<170>  PatentIn version 3.4

<210>  1
<211>  21
<212>  DNA
<213>  Artificial

<220>
<223>  primer

<400>  1
tgccacactc catcatcaat g                                                21


<210>  2
<211>  20
<212>  DNA
<213>  Artificial

<220>
<223>  primer

<400>  2
cgcaaatgca gatggatcag                                                  20


<210>  3
<211>  30
<212>  DNA
<213>  Artificial

<220>
<223>  primer

<400>  3
accttcccac ttactgaaaa ggtcaaggcc                                       30